# EUROPEAN PATENT SPECIFICATION

(11) **EP 1 940 436 B1**
(45) Date of publication and mention of the grant of the patent: **04.12.2013**
(21) Application number: 06802987.5
(22) Date of filing: 06.09.2006
(51) Int. Cl.: A61K 38/00, A61K 38/10, A61K 39/00, A61K 38/04

(54) **METHODS FOR TREATING IMMUNE MEDIATED NEUROLOGICAL DISEASES**
VERFAHREN ZUR BEHANDLUNG VON IMMUNVERMITTELTEN NEUROLOGISCHEN ERKRANKUNGEN
METHODES DE TRAITEMENT DE MALADIES NEUROLOGIQUES D'ORIGINE IMMUNITAIRE

(30) Priority: 06.09.2005 US 714180 P; 22.02.2006 US 775184 P; 08.03.2006 US 779853 P
(43) Date of publication of application: 09.07.2008
(73) Proprietor: Trinity Therapeutics, Inc., Westlake Village, CA 91361 (US)
(72) Inventor: BODIE, Neil M., Agoura Hills, California 91301 (US); BODIE, Renee, Agoura Hills, California 91301 (US); ALTMAN, Elliot, Athens, Georgia 30605 (US)
(74) Representative: Cornish, Kristina Victoria Joy
(86) International application number: PCT/US2006/034603
(87) International publication number: WO 2007/030475

(56) References cited:
- WO-A2-01/45746
- WO-A2-01/45746
- WO-A2-02/38592
- WO-A2-2005/086947
- US-A1- 2003 204 050
- DELANO ET AL.: 'Convergent Solutions to Binding at a Protein-Protein Interface' SCIENCE vol. 287, February 2000, pages 1279 - 1283, XP000985914
- VERKHIVKER ET AL.: 'Monte Carlo Simulations of the Peptide Recognition at the Consensus Binding Site of the Constant Fragment of Human Immunoglobulin G: the Energy Landscape Analysis of a Hot Spot at the Intermolecular Interface' PROTEINS: STUCTURE, FUNCTION, AND GENETICS vol. 48, 2002, pages 539 - 557, XP003009811

## Description

### TECHNICAL FIELD

This document relates to inhibition of immune complex formation, and more particularly to inhibition of immune complex formation by polypeptides and other small molecules.

### BACKGROUND

Humoral immune responses are triggered when an antigen binds specifically to an antibody. The combination of an antibody molecule and an antigen forms a small, relatively soluble immune complex. Antigens either can be foreign substances, such as viral or bacterial polypeptides, or can be "self-antigens" such as polypeptides normally found in the human body. The immune system normally distinguishes foreign antigens from self-antigens. "Autoimmune" disease can occur, however, when this system breaks down, such that the immune system turns upon the body and destroys tissues or organ systems as if they were foreign substances. Larger immune complexes are more pathogenic than small, more soluble immune complexes. The formation of large, relatively insoluble immune complexes can result from both the interaction of antibody molecules with antigen and the interaction of antibody molecules with each other. Such immune complexes also can result from interactions between antibodies in the absence of antigen.

Antibodies can prevent infections by coating viruses or bacteria, but otherwise are relatively harmless by themselves. In contrast, organ specific tissue damage can occur when antibodies combine with antigens and the resulting immune complexes bind to certain effector molecules in the body. Effector molecules are so named because they carry out the pathogenic effects of immune complexes. By inhibiting the formation of large, insoluble immune complexes, or by inhibiting the binding of immune complexes to effector molecules, the tissue damaging effects of immune complexes could be prevented.

WO 01/45746 describes methods and compositions for prolonging elimination half-times of bioactive compounds.

WO 2005/086947 describes methods for inhibiting immune complex formation, particularly immune complex formation associated with rheumatoid arthritis.

US 2003/0204050 describes inhibition of immune complex formation.

### SUMMARY

The invention is as set out in the claims.

This document is based on the discovery that polypeptides having amino acid sequences such as those set forth in SEQ ID NOS:2 and 16 can bind specifically and with high affinity to the C_{H}2-C_{H}3 domain of an immunoglobulin molecule, thus inhibiting the formation of insoluble immune complexes containing antibodies and antigens, and preventing the binding of such complexes to effector molecules. This document provides such polypeptides for use in inhibiting immune complex formation associated with amyotrophic lateral sclerosis (ALS) or Alzheimer's disease (AD).

This document describes a method for inhibiting immune complex formation in a subject. The method can include administering to the subject a composition comprising a purified polypeptide, wherein the polypeptide comprises the amino acid sequence (Xaa₁)ₙ-Cys-Ala-Xaa₂-His-Leu-Gly-Glu-Leu-Val-Trp-Cys-Thr-(Xaa₃)ₙ (SEQ ID NO:35), wherein Xaa₁ is any amino acid, Xaa₂ is Arg, Trp, 5-HTP, Tyr, or Phe, Xaa₃ is any amino acid, and n is 0, 1, 2, 3, 4, or 5. The immune complex formation can be associated with amyotrophic lateral sclerosis (ALS). The polypeptide can inhibit binding of ALS IgG Fc to FcγI, FcγIIa, FcγIIb, FcγIIIa, FcγIIIb, FcRn, mC1q, sC1q, wild type SOD1, or mutant SOD1. The method can further include the step of monitoring said subject for a clinical or molecular characteristic of ALS. The monitoring can include electromyography or measuring CNS MCP-I levels, motor neuron immunoglobulin mediated calcium increase, neurotransmitter release, or neuronal cell damage or cell death. The immune complex formation can be associated with Parkinson's disease (PD). The polypeptide can inhibit binding of PD IgG Fc to FcγI, FcγIIa, FcγIIb, FcγIIIa, FcγIIIb, FcRn, mC1q, sC1q, α-synuclein, or aggregates of α-synuclein and microtubules. The method can further include the step of monitoring the subject for a clinical or molecular characteristic of PD. The clinical or molecular characteristic of PD can be a decrease in MCP-1 in the substantia nigra area or increased survival of TH+ cells in the substantia nigra. The immune complex formation can be associated with Alzheimer's disease (AD). The polypeptide can inhibit binding of AD IgG Fc to FcγI, FcγIIa, FcγIIb, FcγIIIa, FcγIIIb, FcRn, mC1q, sClq, β-amyloid peptide, tau protein, microtubules, or aggregates of tau proteins and microtubules. The method can further include the step of monitoring said subject for clinical or molecular characteristics of AD.

The polypeptide can further include a terminal-stabilizing group. The terminal stabilizing group can be at the amino terminus of said polypeptide and can be a tripeptide having the amino acid sequence Xaa-Pro-Pro, wherein Xaa is any amino acid (e.g., Ala). The terminal stabilizing group can be at the carboxy terminus of said polypeptide and can be a tripeptide having the amino acid sequence Pro-Pro-Xaa, wherein Xaa is any amino acid (e.g., Ala). The polypeptide can further include an Asp at the amino terminus of said amino acid sequence.

The polypeptide can have a length of about 10 to about 50 amino acids. The polypeptide can have the amino acid sequence Asp-Cys-Ala-Trp-His-Leu-Gly-Glu-Leu-Val-Trp-Cys-Thr (SEQ ID NO:2), or the amino acid sequence Ala- Pro-Pro-Asp-Cys-Ala-Trp-His-Leu-Gly-Glu-Leu-Val-Trp-Cys-Thr (SEQ ID NO:16).

This document describes a purified polypeptide, the amino acid sequence of which consists of: (Xaa₁)ₙ-Cys-Ala-Xaa₂-His-Leu-Gly-Glu-Leu-Val-Trp-Cys-Thr-(Xaa₃)ₙ (SEQ ID NO:35), wherein Xaa₁ is any amino acid, Xaa₂ is Arg, Trp, 5-HTP, Tyr, or Phe, Xaa₃ is any amino acid, and n is 0, 1, 2, 3, 4, or 5.

This document also describes a method of designing a ligand having specific binding affinity for the C_{H}2-C_{H}3 cleft of an immunoglobulin molecule having bound antigen. The method can include: a) providing data to a computer, the data comprising the atomic coordinates of the amino acid residues at positions 252, 253, 435, and 436 within the C_{H}2-C_{H}3 cleft, and the computer having a computer program capable of generating an atomic model of a molecule from the atomic coordinate data; b) generating with the computer an atomic model of the CH2-CH3 cleft; c) providing to the computer data comprising the atomic coordinates of a candidate compound; d) generating with the computer an atomic model of the candidate compound optimally positioned in the C_{H}2-C_{H}3 cleft; e) determining whether the optimally positioned candidate compound interacts with the amino acid residues within the C_{H}2-C_{H}3 cleft; and f) identifying the candidate compound as a ligand having specific binding affinity for the C_{H}2-C_{H}3 cleft if the candidate compound interacts with the amino acid residues. The ligand can have a binding affinity of at least 1µM (e.g., at least 100 nM or at least 10 nM) for the CH₂ CH₃ cleft. The ligand can be capable of inhibiting the Fc-mediated formation of an immune complex. The ligand can be capable of inhibiting the binding of FcR to the CH₂ CH₃ cleft. The ligand can be capable of inhibiting the binding of C1q to said CH₂ CH₃ cleft. The ligand can be capable of treating ALS, or AD.

In another aspect, this document features the use of a polypeptide in the manufacture of a medicament for treating ALS, or AD, wherein the polypeptide comprises the amino acid sequence (Xaa₁)ₙ-Cys-Ala-Xaa₂-His-Leu-Gly-Glu-Leu-Val-Trp-Cys-Thr-(Xaa₃)ₙ (SEQ ID NO:35), wherein Xaa₁ is any amino acid, Xaa₂ is Arg, Trp, 5-HTP, Tyr, or Phe, Xaa₃ is any amino acid, and n is 0, 1, 2, 3, 4, or 5.

This document also describes a composition comprising a purified polypeptide, the polypeptide comprising the amino acid sequence (Xaa₁)ₙ-Cys-Ala-Xaa₂-His-Leu-Gly-Glu-Leu-Val-Trp-Cys-Thr-(Xaa₃)ₙ (SEQ ID NO:35), wherein Xaa₁ is any amino acid, Xaa₂ is Arg, Trp, 5-HTP, Tyr, or Phe, Xaa₃ is any amino acid, and n is 0, 1, 2, 3, 4, or 5.

Unless otherwise defined, all technical and scientific terms used herein have the same meaning as commonly understood by one of ordinary skill in the art to which this invention pertains. Although methods and materials similar or equivalent to those described herein can be used to practice the invention, suitable methods and materials are described below. In case of conflict, the present specification, including definitions, will control. In addition, the materials, methods, and examples are illustrative only and not intended to be limiting.

The details of one or more embodiments of the invention are set forth in the accompanying drawings and the description below. Other features, objects, and advantages of the invention will be apparent from the description and from the claims.

### DETAILED DESCRIPTION

This document provides polypeptides and other compounds capable of interacting with the C_{H}2-C_{H}3 cleft of an immunoglobulin molecule, such that interaction of the immunoglobulin with other molecules (e.g., effectors or other immunoglobulins) is blocked. Methods for identifying such polypeptides and other compounds also are described, along with compositions and articles of manufacture containing the polypeptides and compounds. In addition, this document provides polypeptides and compounds for use in inhibiting immune complex formation and to treat diseases in which IgG immune complexes bind to effector molecules, such as membrane bound C1q (mClq), soluble C1q (sClq), SOD1, tau protein, α-synuclein, and FcγRs (including, but not limited to FcγRI, FcγRIIa, FcγRIIb, FcγRIIIa, FcγRIIIb, FcRn, and isoforms of FcγRs), which have been shown to be essential mediators of ALS, PD and AD.

### 1. Immunoglobulins

The immunoglobulins make up a class of proteins found in plasma and other bodily fluids that exhibit antibody activity and bind to other molecules (e.g., antigens and certain cell surface receptors) with a high degree of specificity. Based on their structure and biological activity, immunoglobulins can be divided into five classes: IgM, IgG, IgA, IgD, and IgE. IgG is the most abundant antibody class in the body. With the exception of the IgMs, immunoglobulins are composed mainly of four peptide chains that are linked by several intrachain and interchain disulfide bonds. For example, the IgGs are composed of two polypeptide heavy chains (H chains) and two polypeptide light chains (L chains), which are coupled by disulfide bonds and non-covalent bonds to form a protein molecule with a twisted "Y" shape configuration and a molecular weight of approximately 160,000 daltons. The average IgG molecule contains approximately 4.5 interchain disulfide bonds and approximately 12 intrachain disulfide bonds (Frangione and Milstein (1968) J. Mol. Biol. 33:893-906).

The light and heavy chains of immunoglobulin molecules are composed of constant regions and variable regions (see, e.g., Padlan (1994) Mol. Immunol. 31:169-217). For example, the light chains of an IgG1 molecule each contain a variable domain (V_{L}) and a constant domain (C_{L}). The heavy chains each have four domains: an amino terminal variable domain (V_{H}), followed by three constant domains (C_{H}1, C_{H}2, and the carboxy terminal C_{H}3). A hinge region corresponds to a flexible junction between the C_{H}1 and C_{H}2 domains. Papain digestion of an intact IgG molecule results in proteolytic cleavage at the hinge and produces an Fc fragment that contains the C_{H}2 and C_{H}3 domains, and two identical Fab fragments that each contain a C_{H}1, C_{L}, V_{H}, and V_{L} domain. The Fc fragment has complement- and tissue-binding activity, while the Fab fragments have antigen-binding activity.

Immunoglobulin molecules can interact with other polypeptides through various regions. The majority of antigen binding, for example, occurs through the V_{L}/V_{H} region of the Fab fragment. The hinge region also is thought to be important, as immunological dogma states that the binding sites for Fc receptors (FcR) are found in the hinge region of IgG molecules (see, e.g., Raghavan and Bjorkman (1996) Annu. Rev. Dev. Biol. 12:181-200). More recent evidence, however, suggests that FcR interacts with the hinge region primarily when the immunoglobulin is monomeric (i.e., not immune-complexed). Such interactions typically involve the amino acids at positions 234-237 of the Ig molecule (Wiens et al. (2000) J. Immunol. 164:5313-5318).

Immunoglobulin molecules also can interact with other polypeptides through a cleft within the C_{H}2-C_{H}3 domain. The "C_{H}2-C_{H}3 cleft" typically includes the amino acids at positions 251-255 within the C_{H}2 domain and the amino acids at positions 424-436 within the C_{H}3 domain. As used herein, numbering is with respect to an intact IgG molecule as in Kabat et al. (Sequences of Proteins of Immunological Interest, 5th ed., Public Health Service, U.S. Department of Health and Human Services, Bethesda, MD). The corresponding amino acids in other immunoglobulin classes can be readily determined by those of ordinary skill in the art.

The C_{H}2-C_{H}3 cleft is unusual in that it is characterized by both a high degree of solvent accessibility and a predominantly hydrophobic character, suggesting that burial of an exposed hydrophobic surface is an important driving force behind binding at this site. A three-dimensional change occurs at the IgG C_{H}2-C_{H}3 cleft upon antigen binding, allowing certain residues (e.g., a histidine at position 435) to become exposed and available for binding. Direct evidence of three-dimensional structural changes that occur upon antigen binding was found in a study using monoclonal antibodies sensitive to conformational changes in the Fc region of human IgG. Five IgG epitopes were altered by antigen binding: two within the hinge region and three within the C_{H}2-C_{H}3 cleft (Girkontraite et al. (1996) Cancer Biother. Radiopharm. 11:87-96). Antigen binding therefore can be important for determining whether an immunoglobulin binds to other molecules through the hinge or the Fc C_{H}2-C_{H}3 region.

The Fc region can bind to a number of effector molecules and other proteins, including the following:
(1) FcRn - The neonatal Fc receptor determines the half life of the antibody molecule in the general circulation (Leach et al., (1996) J. Immunol. 157:3317-3322; Gheti and Ward (2000) Ann. Rev. Immunol. 18:739-766). Mice genetically lacking FcRn are protected from the deleterious effects of pathogenic autoantibodies due to the shortened half-life of the autoantibodies (Liu et al. (1997) J. Exp. Med. 186:777-783). The only binding site of FcRn to the IgG Fc is the IgG Fc C_{H}2-C_{H}3 cleft and HIS 435 has been shown by 3D structure and alanine scan to be essential to FcRn to IgG Fc binding (Shields et al. (2001) JBC 276:6591-6604 and Martin et al., (2001), Mol Cell, 7:867-877). Since the peptides provided herein bind with high affinity to the C_{H}2-C_{H}3 cleft and HIS 435, the peptides are direct inhibitors of (immune complexed) IgG Fc to FcRn binding. An inhibitor of FcRn binding to immune complexes or to pathogenic autoantibodies would be useful in treating diseases involving pathogenic autoantibodies and/or immune complexes.
(2) FcR - The cellular Fc Receptor provides a link between the humoral immune response and cell-mediated effector systems (Hamano et al. (2000) J. Immunol. 164:6113-6119; Coxon et al. (2001) Immunity 14:693-704; Fossati et al. (2001) Eur. J. Clin. Invest. 31:821-831). The Fcγ Receptors are specific for IgG molecules, and include FcγRI, FcγRIIa, FcγRIIb, and FcγRIII. These isotypes bind with differing affinities to monomeric and immune-complexed IgG.
(3) C1q - The first component of the classical complement pathway is C1, which exists in blood serum as a complex of three proteins, C1q, C1r, and C1s. The classical complement pathway is activated when C1q binds to the Fc regions of antigen-bound IgG or IgM. Although the binding of C1q to a single Fc region is weak, C1q can form tight bonds to a cluster of Fc regions. At this point C1 becomes proteolytically active.

The formation of immune complexes via interactions between immunoglobulin Fc regions and other antibodies or other factors (e.g., those described above) is referred to herein as "Fc-mediated immune complex formation" or "the Fc-mediated formation of an immune complex." Immune complexes containing such interactions are termed "Fc-mediated immune complexes." Fc-mediated immune complexes can include immunoglobulin molecules with or without bound antigen, and typically include C_{H}2-C_{H}3 cleft-specific ligands that have higher binding affinity for immune complexed antibodies than for monomeric antibodies.

### 2. Purified Polypeptides

As used herein, a "polypeptide" is any chain of amino acid residues, regardless of post-translational modification (e.g., phosphorylation or glycosylation). Polypeptides provided herein typically are between 10 and 50 amino acids in length (e.g., 10, 11, 12, 13, 14, 15, 20, 25, 30, 35, 40, 45, or 50 amino acids in length). Polypeptides that are between 10 and 20 amino acids in length (e.g., 10, 11, 12, 13, 14, 15, 16, 17, 18, 19, or 20 amino acids in length) can be particularly useful.

The term "amino acid" refers to natural amino acids, unnatural amino acids, and amino acid analogs, all in their D and L stereoisomers if their structures so allow. Natural amino acids include alanine (Ala), arginine (Arg), asparagine (Asn), aspartic acid (Asp), cysteine (Cys), glutamine (Gln), glutamic acid (Glu), glycine (Gly), histidine (His), isoleucine (Ile), leucine (Leu), lysine (Lys), methionine (Met), phenylalanine (Phe), proline (Pro), serine (Ser), threonine (Thr), tryptophan (Trp), tyrosine (Tyr), and valine(Val). Unnatural amino acids include, but are not limited to azetidinecarboxylic acid, 2-aminoadipic acid, 3-aminoadipic acid, beta-alanine, aminopropionic acid, 2-aminobutyric acid, 4-aminobutyric acid, 6-aminocaproic acid, 2-aminoheptanoic acid, 2-aminoisobutyric acid, 3-aminoisobutyric acid, 2-aminopimelic acid, 2,4-diaminoisobutyric acid, desmosine, 2,2'-diaminopimelic acid, 2,3-diaminopropionic acid, N-ethylglycine, N-ethylasparagine, hydroxylysine, allo-hydroxylysine, 3-hydroxyproline, 4-hydroxyproline, isodesmosine, allo-isoleucine, N-methylglycine, N-methylisoleucine, N-methylvaline, norvaline, norleucine, ornithine, and pipecolic acid.

An "analog" is a chemical compound that is structurally similar to another but differs slightly in composition (as in the replacement of one atom by an atom of a different element or in the presence of a particular functional group). An "amino acid analog" therefore is structurally similar to a naturally occurring amino acid molecule as is typically found in native polypeptides, but differs in composition such that either the C-terminal carboxy group, the N-terminal amino group, or the side-chain functional group has been chemically modified to another functional group. Amino acid analogs include natural and unnatural amino acids which are chemically blocked, reversibly or irreversibly, or modified on their N-terminal amino group or their side-chain groups, and include, for example, methionine sulfoxide, methionine sulfone, S-(carboxymethyl)-cysteine, S-(carboxymethyl)-cysteine sulfoxide and S-(carboxymethyl)-cysteine sulfone. Amino acid analogs may be naturally occurring, or can be synthetically prepared. Non-limiting examples of amino acid analogs include 5-Hydroxytrpophan (5-HTP), aspartic acid-(beta-methyl ester), an analog of aspartic acid; N-ethylglycine, an analog of glycine; and alanine carboxamide, an analog of alanine. Other examples of amino acids and amino acids analogs are listed in Gross and Meienhofer, The Peptides: Analysis, Synthesis, Biology, Academic Press, Inc., New York (1983).

The stereochemistry of a polypeptide can be described in terms of the topochemical arrangement of the side chains of the amino acid residues about the polypeptide backbone, which is defined by the peptide bonds between the amino acid residues and the α-carbon atoms of the bonded residues. In addition, polypeptide backbones have distinct termini and thus direction. The majority of naturally occurring amino acids are L-amino acids. Naturally occurring polypeptides are largely comprised of L-amino acids.

D-amino acids are the enantiomers of L-amino acids and can form peptides that are herein referred to as "inverso" polypeptides (i.e., peptides corresponding to native peptides but made up of D-amino acids rather than L-amino acids). A "retro" polypeptide is made up of L-amino acids, but has an amino acid sequence in which the amino acid residues are assembled in the opposite direction of the native peptide sequence.

"Retro-inverso" modification of naturally occurring polypeptides involves the synthetic assembly of amino acids with α-carbon stereochemistry opposite to that of the corresponding L-amino acids (i.e., D- or D-allo-amino acids), in reverse order with respect to the native polypeptide sequence. A retro-inverso analog thus has reversed termini and reversed direction of peptide bonds, while approximately maintaining the topology of the side chains as in the native peptide sequence. The term "native" refers to any sequence of L-amino acids used as a starting sequence for the preparation of partial or complete retro, inverso or retro-inverso analogs.

Partial retro-inverso polypeptide analogs are polypeptides in which only part of the sequence is reversed and replaced with enantiomeric amino acid residues. Since the retro-inverted portion of such an analog has reversed amino and carboxyl termini, the amino acid residues flanking the retro-inverted portion can be replaced by side-chain-analogous α-substituted geminal-diaminomethanes and malonates, respectively. Alternatively, a polypeptide can be a complete retro-inverso analog, in which the entire sequence is reversed and replaced with D-amino acids.

The amino acid sequences of the polypeptides provided herein are somewhat constrained, but can have some variability. For example, the polypeptides provided herein typically include the amino acid sequence Xaa₁-Cys-Ala-Xaa₂-His-Xaa₃-Xaa₄-Xaa₅-Leu-Val-Trp-Cys-Xaa₆ (SEQ ID NO:1), wherein the residues denoted by Xaaₙ can display variability. For example, Xaa₁ can be absent or can be any amino acid (e.g., Arg or Asp). Xaa₂ can be any amino acid, such as Phe, Tyr, Trp, Arg, 5-hydoxytryptophan (5-HTP), or any other amino acid derivative. Xaa₃ can be any amino acid. Xaa₄ can be Gly or Ala, while Xaa₅ can be Glu or Ala. Like Xaa₁, Xaa₆ also can be absent or can be any amino acid.

In some embodiments, a polypeptide can include the amino acid sequence Asp-Cys-Ala-Trp-His-Leu-Gly-Glu-Leu-Val-Trp-Cys-Thr (SEQ ID NO:2). Alternatively, a polypeptide can include the amino acid sequence Asp-Cys-Ala-Phe-His-Leu-Gly-Glu-Leu-Val-Trp-Cys-Thr (SEQ ID NO:3) or Asp-Cys-Ala-Arg-His-Leu-Gly-Glu-Leu-Val-Trp-Cys-Thr (SEQ ID NO:4). In some embodiments, a polypeptide can include the amino acid sequence Arg-Cys-Ala-Arg-His-Leu-Gly-Glu-Leu-Val-Trp-Cys-Thr (SEQ ID NO: 5), Arg-Cys-Ala-Trp-His-Leu-Gly-Glu-Leu-Val-Trp-Cys-Thr (SEQ ID NO: 6), or Arg-Cys-Ala-Phe-His-Leu-Gly-Glu-Leu-Val-Trp-Cys-Thr (SEQ ID NO:7).

In some embodiments, a polypeptide can include the amino acid sequence Cys-Ala-Xaa-His-Leu-Gly-Glu-Leu-Val-Trp-Cys (SEQ ID NO:8), in which Xaa can be Phe, Tyr, Trp, Arg, or 5-HTP. For example, polypeptides can include the following amino acid sequences: Cys-Ala-Phe-His-Leu-Gly-Glu-Leu-Val-Trp-Cys-Thr (SEQ ID NO:9), Cys-Ala-Trp-His-Leu-Gly-Glu-Leu-Val-Trp-Cys-Thr (SEQ ID NO:10), and Cys-Ala-Arg-His-Leu-Gly-Glu-Leu-Val-Trp-Cys-Thr (SEQ ID NO:11).

The polypeptides provided herein can be modified for use *in vivo* by the addition, at the amino- or carboxy-terminal end, of a stabilizing agent to facilitate survival of the polypeptide *in vivo*. This can be useful in situations in which peptide termini tend to be degraded by proteases prior to cellular uptake. Such blocking agents can include, without limitation, additional related or unrelated peptide sequences that can be attached to the amino- and/or carboxy-terminal residues of the polypeptide (e.g., an acetyl group attached to the N-terminal amino acid or an amide group attached to the C-terminal amino acid). Such attachment can be achieved either chemically, during the synthesis of the polypeptide, or by recombinant DNA technology using methods familiar to those of ordinary skill in the art. Alternatively, blocking agents such as pyroglutamic acid or other molecules known in the art can be attached to the amino- and/or carboxy-terminal residues, or the amino group at the amino terminus or the carboxy group at the carboxy terminus can be replaced with a different moiety.

A proline or an Xaa-Pro-Pro (e.g., Ala-Pro-Pro) sequence at the amino terminus can be particularly useful (see, e.g., WO 00/22112). For example, a polypeptide can include the amino acid sequence Xaa₁-Pro-Pro-Cys-Ala-Xaa₂-His-Leu-Gly-Glu-Leu-Val-Trp-Cys-Thr (SEQ ID NO:12), where Xaa₁ is any amino acid (e.g., Ala), and Xaa₂ is Trp, Tyr, Phe, Arg, or 5-HTP. Thus, for example, a polypeptide can include the amino acid sequence Xaa₁-Pro-Pro-Cys-Ala-Trp-His-Leu-Gly-Glu-Leu-Val-Trp-Cys-Thr (SEQ ID NO:13), Xaa₁-Pro-Pro-Cys-Ala-Arg-His-Leu-Gly-Glu-Leu-Val-Trp-Cys-Thr or Xaa₁-Pro-Pro-Cys-Ala-Phe-His-Leu-Gly-Glu-Leu-Val-Trp-Cys-Thr (SEQ ID NO: 15). Alternatively, a polypeptide can include the amino acid sequence Xaa₁-Pro-Pro-Asp-Cys-Ala-Trp-His-Leu-Gly-Glu-Leu-Val-Trp-Cys-Thr,

Xaa₁-Pro-Pro-Asp-Cys-Ala-Arg-His-Leu-Gly-Glu-Leu-Val-Trp-Cys-Thr (SEQ ID NO:17), Xaa₁-Pro-Pro-Asp-Cys-Ala-Phe-His-Leu-Gly-Glu-Leu-Val-Trp-Cys-Thr (SEQ ID NO:18), Xaa₁-Pro-Pro-Arg-Cys-Ala-Trp-His-Leu-Gly-Glu-Leu-Val-Trp-Cys-Thr (SEQ ID NO:19), Xaa₁-Pro-Pro-Arg-Cys-Ala-Arg-His-Leu-Gly-Glu-Leu-Val-Trp-Cys-Thr (SEQ ID NO:20), or Xaa₁-Pro-Pro-Arg-Cys-Ala-Phe-His-Leu-Gly-Glu-Leu-Val-Trp-Cys-Thr (SEQ ID NO: 21).

The polypeptides provided herein can have a Pro-Pro-Xaa (e.g., Pro-Pro-Ala) sequence at their carboxy termini. For example, a polypeptide can include the amino acid sequence Cys-Ala-Trp-His-Leu-Gly-Glu-Leu-Val-Trp-Cys-Thr-Pro-Pro-Xaa (SEQ ID NO:22), Cys-Ala-Arg-His-Leu-Gly-Glu-Leu-Val-Trp-Cys-Thr-Pro-Pro-Xaa (SEQ ID NO:23), Cys-Ala-Phe-His-Leu-Gly-Glu-Leu-Val-Trp-Cys-Thr-Pro-Pro-Xaa (SEQ ID NO: 24), Asp-Cys-Ala-Trp-His-Leu-Gly-Glu-Leu-Val-Trp-Cys-Thr-Pro-Pro-Xaa (SEQ ID NO:25), Asp-Cys-Ala-Arg-His-Leu-Gly-Glu-Leu-Val-Trp-Cys-Thr-Pro-Pro-Xaa (SEQ ID NO:26), Asp-Cys-Ala-Phe-His-Leu-Gly-Glu-Leu-Val-Trp-Cys-Thr-Pro-Pro-Xaa (SEQ ID NO:27), Arg-Cys-Ala-Trp-His-Leu-Gly-Glu-Leu-Val-Trp-Cys-Thr-Pro- Pro-Xaa (SEQ ID NO:28), Arg-Cys-Ala-Arg-His-Leu-Gly-Glu-Leu-Val-Trp-Cys-Thr-Pro-Pro-Xaa (SEQ ID NO:29), or Arg-Cys-Ala-Phe-His-Leu-Gly-Glu-Leu-Val-Trp-Cys-Thr-Pro-Pro-Xaa (SEQ ID NO:30), wherein Xaa can be any amino acid.

In some embodiments, the polypeptides provided herein can include additional amino acid sequences at the amino terminus of the sequence set forth in SEQ ID NO:1, the carboxy terminus of the sequence set forth in SEQ ID NO:1, or both. For example, a polypeptide can contain the amino acid sequence Trp-Glu-Ala-Xaa₁-Cys-Ala-Xaa₂-His-Xaa₃-Xaa₄-Xaa₅-Leu-Val-Trp-Cys-Xaa₆-Lys-Val-Glu-Glu (SEQ ID NO:31), wherein the residues denoted by Xaaₙ can display variability. As for the amino acid sequence set forth in SEQ ID NO: 1, Xaa₁ can be absent or can be any amino acid (e.g., Arg or Asp); Xaa₂ can be Phe, Tyr, 5-HTP, Trp, or Arg; Xaa₃ can be any amino acid; Xaa₄ can be Gly or Ala; Xaa₅ can be Glu or Ala; and Xaa₆ can be absent or can be any amino acid. In one embodiment, a polypeptide can include the amino acid sequence Trp-Glu-Ala-Asp-Cys-Ala-Xaa-His-Leu-Gly-Glu-Leu-Val-Trp-Cys-Thr-Lys-Val-Glu-Glu (SEQ ID NO:32), where Xaa is Arg, Trp, 5-HTP, Tyr, or Phe. For example, a polypeptide can include the amino acid sequence Trp-Glu-Ala-Asp-Cys-Ala-Trp-His-Leu-Gly-Glu-Leu-Val-Trp-Cys-Thr-Lys-Val-Glu-Glu (SEQ ID NO:33).

In some embodiments, a polypeptide can consist of the amino acid sequence (Xaa₁)ₙ-Xaa₂-Cys-Ala-Xaa₃-His-Xaa₄-Xaa₅-Xaa₆-Leu-Val-Trp-Cys-(Xaa₇)ₙ (SEQ ID NO:34), wherein the residues denoted by Xaa can display variability, and n can be an integer from 0 to 10 (e.g., 0, 1, 2, 3, 4, 5, 6, 7, 8, 9, or 10). For example, Xaa₁ can be any amino acid; Xaa₂ can be absent or can be any amino acid (e.g., Arg or Asp); Xaa₃ can be Phe, Tyr, Trp, Arg, or 5-HTP; Xaa₄ can be any amino acid; Xaa₅ can be Gly or Ala; Xaa₆ can be Glu or Ala; Xaa₇ can be any amino acid; and n can be from 0 to 5 (e.g., 0, 1, 2, 3, 4, or 5). Alternatively, a polypeptide can consist of the amino acid sequence (Xaa₁)ₙ-Cys-Ala-Xaa₂-His-Leu-Gly-Glu-Leu-Val-Trp-Cys-Thr-(Xaa₃)ₙ (SEQ ID NO:35), wherein Xaa₁ is any amino acid, Xaa₂ is Phe, Trp, Tyr, Arg, or 5-HTP, Xaa₃ is any amino acid, and n is an integer from 0 to 5 (e.g., 0, 1, 2, 3, 4, or 5). Examples of polypeptides within these embodiments include, without limitation, polypeptides consisting of the amino acid sequence Ala-Ala-Ala-Ala-Ala-Asp-Cys-Ala-Arg-His-Leu-Gly-Glu-Leu-Val-Trp-Cys-Ala-Ala-Ala-Ala-Ala (SEQ ID NO:36), Ala-Ala-Arg-Cys-Ala-Arg-His-Leu-Gly-Glu-Leu-Val-Trp-Cys-Thr-Ala-Ala(SEQ ID NO:37), or Ala-Ala-Ala-Asp-Cys-Ala-Phe-Trp-His-Leu-Gly-Glu-Leu-Val-Trp-Cys-Thr-Ala-Ala (SEQ ID NO:38).

The amino acid sequences set forth in SEQ ID NOs:1-38 typically contain two cysteine residues. Polypeptides containing these amino acid sequences can cyclize due to formation of a disulfide bond between the two cysteine residues. A person having ordinary skill in the art can use, for example, Ellman's Reagent to determine whether a peptide containing multiple cysteine residues is cyclized. In some embodiments, these cysteine residues can be substituted with other natural or non-natural amino acid residues that can form lactam bonds rather than disulfide bonds. For example, one cysteine residue could be replaced with aspartic acid or glutamic acid, while the other could be replaced with ornithine or lysine. Any of these combinations could yield a lactam bridge. By varying the amino acids that form a lactam bridge, a polypeptide provided herein can be generated that contains a bridge approximately equal in length to the disulfide bond that would be formed if two cysteine residues were present in the polypeptide.

The polypeptides provided herein can contain an amino acid tag. A "tag" is generally a short amino acid sequence that provides a ready means of detection or purification through interactions with an antibody against the tag or through other compounds or molecules that recognize the tag. For example, tags such as c-myc, hemagglutinin, polyhistidine, or FLAG^{®} can be used to aid purification and detection of a polypeptide. As an example, a polypeptide with a polyhistidine tag can be purified based on the affinity of histidine residues for nickel ions (e.g., on a Ni-NTA column), and can be detected in western blots by an antibody against polyhistidine (e.g., the Penta-His antibody; Qiagen, Valencia, CA). Tags can be inserted anywhere within the polypeptide sequence, although insertion at the amino- or carboxy-terminus is particularly useful.

Also provided herein are peptidomimetic compounds designed on the basis of the amino acid sequences of polypeptides. Peptidomimetic compounds are synthetic, non-peptide compounds having a three-dimensional conformation (i.e., a "peptide motif,") that is substantially the same as the three-dimensional conformation of a selected peptide, and can thus confer the same or similar function as the selected peptide. Peptidomimetic compounds provided herein can be designed to mimic any of the polypeptides described herein.

Peptidomimetic compounds that are protease resistant are particularly useful. Furthermore, peptidomimetic compounds may have additional characteristics that enhance therapeutic utility, such as increased cell permeability and prolonged biological half-life. Such compounds typically have a backbone that is partially or completely non-peptide, but with side groups that are identical or similar to the side groups of the amino acid residues that occur in the peptide upon which the peptidomimetic compound is based. Several types of chemical bonds (e.g., ester, thioester, thioamide, retroamide, reduced carbonyl, dimethylene and ketomethylene) are known in the art to be useful substitutes for peptide bonds in the construction of peptidomimetic compounds.

The interactions between a polypeptide as provided herein and an immunoglobulin molecule typically occur through the C_{H}2-C_{H}3 cleft of the immunoglobulin. Such interactions are engendered through physical proximity and are mediated by, for example, hydrophobic interactions. The "binding affinity" of a polypeptide for an immunoglobulin molecule refers to the strength of the interaction between the polypeptide and the immunoglobulin. Binding affinity typically is expressed as an equilibrium dissociation constant (K_{d}), which is calculated as K_{d} = k_{off}/kₒₙ, where k_{off} = the kinetic dissociation constant of the reaction, and kₒₙ = the kinetic association constant of the reaction. K_{d} is expressed as a concentration, with a low K_{d} value (e.g., less than 100 nM) signifying high affinity. Polypeptides that can interact with an immunoglobulin molecule typically have a binding affinity of at least 1 µM (e.g., at least 500 nM, at least 100 nM, at least 50 nM, or at least 10 nM) for the C_{H}2-C_{H}3 cleft of the immunoglobulin.

Polypeptides provided herein can bind with substantially equivalent affinity to immunoglobulin molecules that are bound by antigen and to monomeric immunoglobulins. Alternatively, polypeptides can have a higher binding affinity (e.g., at least 10-fold, at least 100-fold, or at least 1000-fold higher binding affinity) for immunoglobulin molecules that are bound by antigen than for monomeric immunoglobulins. Conformational changes that occur within the Fc region of an immunoglobulin molecule upon antigen binding to the Fab region are likely involved in a difference in affinity. The crystal structures of bound and unbound NC6.8 Fab (from a murine monoclonal antibody) showed that the tail of the Fab heavy chain was displaced by 19 angstroms in crystals of the antigen/antibody complex, as compared to its position in unbound Fab (Guddat et al. (1994) J. Mol. Biol. 236-247-274). Since the C-terminal tail of the Fab region is connected to the Fc region in an intact antibody, this shift would be expected to affect the conformation of the C_{H}2-C_{H}3 cleft. Furthermore, examination of several three-dimensional structures of intact immunoglobulins revealed a direct physical connection between the Fab heavy chain and the Fc C_{H}2-C_{H}3 cleft (Harris et al. (1997) Biochemistry 36:1581-1597; Saphire et al. (2001) Science 293:1155-1159).

Molecular modeling of the C_{H}2-C_{H}3 cleft of monomeric (i.e., unbound) and immune-complexed IgG reveal that the monomeric Fc C_{H}2-C_{H}3 cleft has a closed configuration, which can prevent binding to critical amino acid residues (e.g., His 435; see, for example, O'Brien et al. (1994) Arch. Biochem. Biophys. 310:25-31; Jefferies et al. (1984) Immunol. Lett. 7:191-194; and West et al. (2000) Biochemistry 39:9698-9708). Immune-complexed (antigen-bound) IgG, however, has a more open configuration and thus is more conducive to ligand binding. The binding affinity of RF for immune-complexed IgG, for example, is much greater than the binding affinity of RF for monomeric IgG (Corper et al. (1997) Nat. Struct. Biol. 4:374; Sohi et al. (1996) Immunol. 88:636). The same typically is true for polypeptides provided herein.

Because the polypeptides provided herein can bind to the C_{H}2-C_{H}3 cleft of immunoglobulin molecules, they can be useful for blocking the interaction of other factors (e.g., FcRn, FcR, C1q, histones, MBP, SOD1 and other immunoglobulins) to the Fc region of the immunoglobulin, and thus can inhibit Fc-mediated immune complex formation. By "inhibit" is meant that Fc-mediated immune complex formation is reduced in the presence of a polypeptide, as compared to the level of immune complex formation in the absence of the polypeptide. Such inhibiting can occur *in vitro* (e.g., in a test tube) or *in vivo* (e.g., in an individual). Any suitable method can be used to assess the level of immune complex formation. Many such methods are known in the art, and some of these are described herein.

Polypeptides provided herein typically interact with the C_{H}2-C_{H}3 cleft of an immunoglobulin molecule in a monomeric fashion (i.e., interact with only one immunoglobulin molecule and thus do not link two or more immunoglobulin molecules together) with a 1:2 IgG Fc to peptide stoichiometry. Interactions with other immunoglobulin molecules through the Fc region therefore are precluded by the presence of the polypeptide. The inhibition of Fc-mediated immune complex formation can be assessed *in vitro,* for example, by incubating an IgG molecule with a labeled immunoglobulin molecule (e.g., a fluorescently or enzyme (ELISA) labeled Fc Receptor or C1q in the presence and absence of a polypeptide described herein, and measuring the amount of labeled immunoglobulin that is incorporated into an immune complex. Other methods suitable for detecting immune complex formation also may be used, as discussed below.

### 3. Preparation and Purification of Polypeptides

The polypeptides provided herein can be produced by a number of methods, many of which are well known in the art. By way of example and not limitation, a polypeptide can be obtained by extraction from a natural source (e.g., from isolated cells, tissues or bodily fluids), by expression of a recombinant nucleic acid encoding the polypeptide (as, for example, described below), or by chemical synthesis (e.g., by solid-phase synthesis or other methods well known in the art, including synthesis with an ABI peptide synthesizer; Applied Biosystems, Foster City, CA). Methods for synthesizing retro-inverso polypeptide analogs (Bonelli et al. (1984) Int. J. Peptide Protein Res. 24:553-556; and Verdini and Viscomi (1985) J. Chem. Soc. Perkin Trans. I:697-701), and some processes for the solid-phase synthesis of partial retro-inverso peptide analogs also have been described (see, for example, European Patent number EP0097994).

This document provides isolated nucleic acid molecules encoding the polypeptides described herein. As used herein, "nucleic acid" refers to both RNA and DNA, including cDNA, genomic DNA, and synthetic (e.g., chemically synthesized) DNA. The nucleic acid can be double-stranded or single-stranded (i.e., a sense or an antisense single strand).

The term "isolated" as used herein with reference to a nucleic acid refers to a naturally- occurring nucleic acid that is not immediately contiguous with both of the sequences with which it is immediately contiguous (one at the 5' end and one at the 3' end) in the naturally-occurring genome of the organism from which it is derived. The term "isolated" as used herein with respect to nucleic acids also includes any non-naturally-occurring nucleic acid sequence, since such non-naturally-occurring sequences are not found in nature and do not have immediately contiguous sequences in a naturally-occurring genome.

An isolated nucleic acid can be, for example, a DNA molecule, provided one of the nucleic acid sequences that is normally immediately contiguous with the DNA molecule in a naturally-occurring genome is removed or absent. Thus, an isolated nucleic acid includes, without limitation, a DNA molecule that exists as a separate molecule (e.g., a chemically synthesized nucleic acid, or a cDNA or genomic DNA fragment produced by PCR or restriction endonuclease treatment) independent of other sequences as well as DNA that is incorporated into a vector, an autonomously replicating plasmid, a virus (e.g., a retrovirus, lentivirus, adenovirus, or herpes virus), or into the genomic DNA of a prokaryote or eukaryote. In addition, an isolated nucleic acid can include an engineered nucleic acid such as a recombinant DNA molecule that is part of a hybrid or fusion nucleic acid. A nucleic acid existing among hundreds to millions of other nucleic acids within, for example, cDNA libraries or genomic libraries, or gel slices containing a genomic DNA restriction digest, is not considered an isolated nucleic acid.

Also provided are vectors containing the nucleic acids described herein. As used herein, a "vector" is a replicon, such as a plasmid, phage, or cosmid, into which another DNA segment may be inserted so as to bring about the replication of the inserted segment. The vectors provided herein are preferably expression vectors, in which the nucleotides encode the polypeptides with an initiator methionine, operably linked to expression control sequences. As used herein, "operably linked" means incorporated into a genetic construct so that expression control sequences effectively control expression of a coding sequence of interest. An "expression control sequence" is a DNA sequence that controls and regulates the transcription and translation of another DNA sequence, and an "expression vector" is a vector that includes expression control sequences, so that a relevant DNA segment incorporated into the vector is transcribed and translated. A coding sequence is "operably linked" and "under the control" of transcriptional and translational control sequences in a cell when RNA polymerase transcribes the coding sequence into mRNA, which then is translated into the protein encoded by the coding sequence.

Methods well known to those skilled in the art may be used to subclone isolated nucleic acid molecules encoding polypeptides of interest into expression vectors containing relevant coding sequences and appropriate transcriptional/translational control signals. See, for example, Sambrook et al., Molecular Cloning: A Laboratory Manual (2nd edition), Cold Spring Harbor Laboratory, New York (1989); and Ausubel et al., Current Protocols in Molecular Biology, Green Publishing Associates and Wiley Interscience, New York (1989). Expression vectors can be used in a variety of systems (e.g., bacteria, yeast, insect cells, and mammalian cells), as described herein. Examples of suitable expression vectors include, without limitation, plasmids and viral vectors derived from, for example, herpes viruses, retroviruses, vaccinia viruses, adenoviruses, and adeno-associated viruses. A wide variety of suitable expression vectors and systems are commercially available, including the pET series of bacterial expression vectors (Novagen, Madison, WI), the Adeno-X expression system (Clontech), the Baculogold baculovirus expression system (BD Biosciences Pharmingen, San Diego, CA), and the pCMV-Tag vectors (Stratagene, La Jolla, CA).

Expression vectors that encode the polypeptides described herein can be used to produce the polypeptides. Expression systems that can be used for small or large scale production of polypeptides include, but are not limited to, microorganisms such as bacteria (e.g., *E. coli* and *B. subtilis*) transformed with recombinant bacteriophage DNA, plasmid DNA, or cosmid DNA expression vectors containing the nucleic acid molecules described herein; yeast (e.g., S. *cerevisiae*) transformed with recombinant yeast expression vectors containing the nucleic acid molecules described herein; insect cell systems infected with recombinant virus expression vectors (e.g., baculovirus) containing the nucleic acid molecules described herein; plant cell systems infected with recombinant virus expression vectors (e.g., tobacco mosaic virus) or transformed with recombinant plasmid expression vectors (e.g., Ti plasmid) containing the nucleic acid molecules described herein; or mammalian cell systems (e.g., primary cells or immortalized cell lines such as COS cells, CHO cells, HeLa cells, HEK 293 cells, and 3T3 L1 cells) harboring recombinant expression constructs containing promoters derived from the genome of mammalian cells (e.g., the metallothionein promoter) or from mammalian viruses (e.g., the adenovirus late promoter and the cytomegalovirus promoter), along with the nucleic acids described herein.

The term "purified polypeptide" as used herein refers to a polypeptide that either has no naturally occurring counterpart (e.g., a peptidomimetic), or has been chemically synthesized and is thus uncontaminated by other polypeptides, or that has been separated or purified from other cellular components by which it is naturally accompanied (e.g., other cellular proteins, polynucleotides, or cellular components). Typically, the polypeptide is considered "purified" when it is at least 70%, by dry weight, free from the proteins and naturally occurring organic molecules with which it naturally associates. A preparation of a purified polypeptide therefore can be, for example, at least 80%, at least 90%, or at least 99%, by dry weight, the polypeptide. Suitable methods for purifying the polypeptides provided herein can include, for example, affinity chromatography, immunoprecipitation, size exclusion chromatography, and ion exchange chromatography. The extent of purification can be measured by any appropriate method, including but not limited to: column chromatography, polyacrylamide gel electrophoresis, or high-performance liquid chromatography.

### 4. Methods of modeling, designing, and identifying compounds

This document also provides methods for designing, modeling, and identifying compounds that can bind to the C_{H}2-C_{H}3 cleft of an immunoglobulin molecule and thus serve as inhibitors of Fc-mediated immune complex formation. Such compounds also are referred to herein as "ligands." Compounds designed, modeled, and identified by methods provided herein typically can interact with an immunoglobulin molecule through the C_{H}2-C_{H}3 cleft, and typically have a binding affinity of at least 1 µM (e.g., at least 500 nM, at least 100 nM, at least 50 nM, or at least 10 nM) for the C_{H}2-C_{H}3 cleft of the immunoglobulin. Such compounds generally have higher binding affinity (e.g., at least 10-fold, at least 100-fold, or at least 1000-fold higher binding affinity) for immune-complexed immunoglobulin molecules than for monomeric immunoglobulin molecules.

Compounds provided herein typically interact with the C_{H}2-C_{H}3 cleft of an immunoglobulin molecule in a monomeric fashion (i.e., interact with only one immunoglobulin molecule and thus do not link two or more immunoglobulin molecules together). The interactions between a compound and an immunoglobulin molecule typically involve the amino acid residues at positions 252, 253, 435, and 436 of the immunoglobulin (number according to Kabat, *supra*). The interaction between compounds described herein and the C_{H}2-C_{H}3 cleft renders the compounds capable of inhibiting the Fc-mediated formation of immune complexes by blocking the binding of other factors (e.g., FcRs, FcRn, histones, MBP, RF, tau protein, α-synuclein, SOD1, and C1q) to the C_{H}2-C_{H}3 cleft.

Compounds identified by methods provided herein can be polypeptides such as, for example, those described herein. Alternatively, a compound can be any suitable type of molecule that can specifically bind to the C_{H}2-C_{H}3 cleft of an immunoglobulin molecule.

By "modeling" is meant quantitative and/or qualitative analysis of receptor-ligand structure/function based on three-dimensional structural information and receptor-ligand interaction models. This includes conventional numeric-based molecular dynamic and energy minimization models, interactive computer graphic models, modified molecular mechanics models, distance geometry and other structure-based constraint models. Modeling typically is performed using a computer and may be further optimized using known methods.

Methods of designing ligands that bind specifically (i.e., with high affinity) to the C_{H}2-C_{H}3 cleft of an immunoglobulin molecule having bound antigen typically are computer-based, and involve the use of a computer having a program capable of generating an atomic model. Computer programs that use X-ray crystallography data are particularly useful for designing ligands that can interact with an Fc C_{H}2-C_{H}3 cleft. Programs such as RasMol, for example, can be used to generate a three dimensional model of a C_{H}2-C_{H}3 cleft and/or determine the structures involved in ligand binding. Computer programs such as INSIGHT (Accelrys, Burlington, MA), GRASP (Anthony Nicholls, Columbia University), Dock (Molecular Design Institute, University of California at San Francisco), and Auto-Dock (Accelrys) allow for further manipulation and the ability to introduce new structures.

Methods can include, for example, providing to a computer the atomic structural coordinates for amino acid residues within the C_{H}2-C_{H}3 cleft (e.g., amino acid residues at positions 252, 253, 435, and 436 of the cleft) of an immunoglobulin molecule in an Fc-mediated immune complex, using the computer to generate an atomic model of the C_{H}2-C_{H}3 cleft, further providing the atomic structural coordinates of a candidate compound and generating an atomic model of the compound optimally positioned within the C_{H}2-C_{H}3 cleft, and identifying the candidate compound as a ligand of interest if the compound interacts with the amino acid residues at positions 252, 253, 435, and 436 of the cleft. The data provided to the computer also can include the atomic coordinates of amino acid residues at positions in addition to 252, 253, 435, and 436. By "optimally positioned" is meant positioned to optimize hydrophobic interactions between the candidate compound and the amino acid residues at positions 252, 253,435, and 436 of the C_{H}2-C_{H}3 cleft.

Alternatively, a method for designing a ligand having specific binding affinity for the C_{H}2-C_{H}3 cleft of an immunoglobulin molecule can utilize a computer with an atomic model of the cleft stored in its memory. The atomic coordinates of a candidate compound then can be provided to the computer, and an atomic model of the candidate compound optimally positioned can be generated. As described herein, a candidate compound can be identified as a ligand having specific binding affinity for the C_{H}2-C_{H}3 cleft of an immunoglobulin molecule if, for example, the compound interacts with the amino acid residues at positions 252, 253, 435, and 436 of the cleft. Monomeric (non-antigen bound) IgG Fc bind at a site distinct from the IgG Fc C_{H}2-C_{H}3 cleft, such as the lower hinge region (Wines et al., 2000, 164:5313-5318) while immune complexed (antigen bound) IgG Fc binding to FcγIIa is inhibited by an IgM rheumatoid factor (RF-AN), which has been shown by 3D structure to only bind to the IgG Fc C_{H}2-C_{H}3 interface cleft (Sohi et al., (1996), Immunology, 88:636-641 and Corper et al., (1997), Nature Structural Biology, 4(5):374-381). Soluble FcγIIa inhibits the binding of immune complexed (but not monomeric, non-immune complexed) IgG Fc to RF-AN (Wines et al., (2003), Immunology, 109:246-254), then inhibitors that bind to the IgG Fc C_{H}2-C_{H}3 cleft, such as the peptides described herein, inhibit the binding of immune complexed (antigen-bound) IgG Fc to FcγRs.

Compounds provided herein also may be interactively designed from structural information of the compounds described herein using other structure-based design/modeling techniques (see, e.g., Jackson (1997) Seminars in Oncology 24:L164-172; and Jones et al. (1996) J. Med. Chem. 39:904-917).

Compounds and polypeptides also can be identified by, for example, characterizing candidate compounds by computer modeling as fitting spatially and preferentially (i.e., with high affinity) into the C_{H}2-C_{H}3 cleft of an immunoglobulin molecule, and then screening those compounds *in vitro* or *in vivo* for the ability to inhibit Fc-mediated immune complex formation. Suitable methods for such *in vitro* and *in vivo* screening include those described herein.

### 5. Compositions and Articles of Manufacture

This document provides methods for treating conditions that arise from abnormal Fc-mediated immune complex formation (e.g., over-production of Fc-mediated immune complexes). In these methods, polypeptides and compounds as described herein can be administered to a subject (e.g., a human or another mammal) having a disease or disorder (e.g., ALS, PD, or AD) that can be alleviated by modulating Fc-mediated immune complex formation and inhibit immune complexed IgG Fc binding to, for example, mClq, sClq, FcγRs, histones, MBP, tau proteins, α-synuclein, SOD1, and FcRn. Typically, one or more polypeptides or compounds can be administered to a subject suspected of having, diagnosed with, or at risk for a disease or condition associated with immune complex formation. A composition can contain one or more polypeptides and compounds described herein. For example, a C_{H}2-C_{H}3 binding polypeptide can be combined with a pharmaceutically acceptable carrier or diluent, and can be administered in an amount and for a period of time that will vary depending upon the nature of the particular disease, its severity, and the subject's overall condition. Typically, a polypeptide can be administered in an inhibitory amount (i.e., in an amount that is effective for inhibiting the production of immune complexes in the cells or tissues contacted by the polypeptide). The polypeptides and methods described herein also can be used prophylactically, e.g., to minimize immunoreactivity in a subject at risk for abnormal or over-production of immune complexes (e.g., a transplant recipient).

The ability of a polypeptide to inhibit Fc-mediated immune complex formation can be assessed by, for example, measuring immune complex levels in a subject before and after treatment. A number of methods can be used to measure immune complex levels in tissues or biological samples, including those that are well known in the art. If the subject is a research animal, for example, immune complex levels in the joints can be assessed by immunostaining following euthanasia. The effectiveness of an inhibitory polypeptide also can be assessed by direct methods such as measuring the level of circulating immune complexes in serum samples. Alternatively, indirect methods can be used to evaluate the effectiveness of polypeptides in live subjects. For example, reduced immune complex formation can be inferred from clinical improvement of immune mediated neurodegenerative diseases or *in vitro* or *in vivo* models of ALS, PD, or AD.

Methods for formulating and subsequently administering therapeutic compositions are well known to those skilled in the art. Dosing is generally dependent on the severity and responsiveness of the disease state to be treated, with the course of treatment lasting from several days to several months, or until a cure is effected or a diminution of the disease state is achieved. Persons of ordinary skill in the art routinely determine optimum dosages, dosing methodologies and repetition rates. Optimum dosages can vary depending on the relative potency of individual polypeptides, and can generally be estimated based on EC50 found to be effective in *in vitro* and *in vivo* animal models. Typically, dosage is from 0.01 µg to 100 g per kg of body weight, and may be given once or more daily, biweekly, weekly, monthly, or even less often. Following successful treatment, it may be desirable to have the patient undergo maintenance therapy to prevent the recurrence of the disease state.

The present document provides pharmaceutical compositions and formulations that include the polypeptides and/or compounds described herein. This document also provides methods for using a polypeptide as described herein in the manufacture of a medicament for reducing immune complex formation (e.g., immune complex formation associated with ALS, PD, or AD). Polypeptides provided herein can be admixed, encapsulated, conjugated or otherwise associated with other molecules, molecular structures, or mixtures of compounds such as, for example, liposomes, polyethylene glycol, receptor targeted molecules, or oral, rectal, topical or other formulations, for assisting in uptake, distribution and/or absorption.

A "pharmaceutically acceptable carrier" (also referred to herein as an "excipient") is a pharmaceutically acceptable solvent, suspending agent, or any other pharmacologically inert vehicle for delivering one or more therapeutic compounds (e.g., C_{H}2-C_{H}3 binding polypeptides) to a subject. Pharmaceutically acceptable carriers can be liquid or solid, and can be selected with the planned manner of administration in mind so as to provide for the desired bulk, consistency, and other pertinent transport and chemical properties, when combined with one or more of therapeutic compounds and any other components of a given pharmaceutical composition. Typical pharmaceutically acceptable carriers that do not deleteriously react with amino acids include, by way of example and not limitation: water; saline solution; binding agents (e.g., polyvinylpyrrolidone or hydroxypropyl methylcellulose); fillers (e.g., lactose and other sugars, gelatin, or calcium sulfate); lubricants (e.g., starch, polyethylene glycol, or sodium acetate); disintegrates (e.g., starch or sodium starch glycolate); and wetting agents (e.g., sodium lauryl sulfate).

The pharmaceutical compositions provided herein can be administered by a number of methods, depending upon whether local or systemic treatment is desired and upon the area to be treated. Administration can be, for example, topical (e.g., transdermal, sublingual, ophthalmic, or intranasal); pulmonary (e.g., by inhalation or insufflation of powders or aerosols); oral; or parenteral (e.g., by subcutaneous, intrathecal, intraventricular, intramuscular, or intraperitoneal injection, or by intravenous drip). Administration can be rapid (e.g., by injection) or can occur over a period of time (e.g., by slow infusion or administration of slow release formulations). For treating tissues in the central nervous system, C_{H}2-C_{H}3 binding polypeptides can be administered by injection or infusion into the cerebrospinal fluid, preferably with one or more agents capable of promoting penetration of the polypeptides across the blood-brain barrier.

Formulations for topical administration of C_{H}2-C_{H}3 binding polypeptides include, for example, sterile and non-sterile aqueous solutions, non-aqueous solutions in common solvents such as alcohols, or solutions in liquid or solid oil bases. Such solutions also can contain buffers, diluents and other suitable additives. Pharmaceutical compositions and formulations for topical administration can include transdermal patches, ointments, lotions, creams, gels, drops, suppositories, sprays, liquids, and powders. Nasal sprays are particularly useful, and can be administered by, for example, a nebulizer or another nasal spray device. Administration by an inhaler also is particularly useful. Conventional pharmaceutical carriers, aqueous, powder or oily bases, thickeners and the like may be necessary or desirable.

Compositions and formulations for oral administration include, for example, powders or granules, suspensions or solutions in water or non-aqueous media, capsules, sachets, or tablets. Such compositions also can incorporate thickeners, flavoring agents, diluents, emulsifiers, dispersing aids, or binders.

Compositions and formulations for parenteral, intrathecal or intraventricular administration can include sterile aqueous solutions, which also can contain buffers, diluents and other suitable additives (e.g., penetration enhancers, carrier compounds and other pharmaceutically acceptable carriers).

Pharmaceutical compositions can include, but are not limited to, solutions, emulsions, aqueous suspensions, and liposome-containing formulations. These compositions can be generated from a variety of components that include, for example, preformed liquids, self-emulsifying solids and self-emulsifying semisolids. Emulsions are often biphasic systems comprising of two immiscible liquid phases intimately mixed and dispersed with each other; in general, emulsions are either of the water-in-oil (w/o) or oil-in-water (o/w) variety. Emulsion formulations have been widely used for oral delivery of therapeutics due to their ease of formulation and efficacy of solubilization, absorption, and bioavailability.

Liposomes are vesicles that have a membrane formed from a lipophilic material and an aqueous interior that can contain the composition to be delivered. Liposomes can be particularly useful due to their specificity and the duration of action they offer from the standpoint of drug delivery. Liposome compositions can be formed, for example, from phosphatidylcholine, dimyristoyl phosphatidylcholine, dipalmitoyl phosphatidylcholine, dimyristoyl phosphatidylglycerol, or dioleoyl phosphatidylethanolamine. Numerous lipophilic agents are commercially available, including LIPOFECTIN^{®} (Invitrogen/Life Technologies, Carlsbad, CA) and EFFECTENE™ (Qiagen, Valencia, CA).

Polypeptides provided herein further encompass any pharmaceutically acceptable salts, esters, or salts of such esters, or any other compound which, upon administration to an animal including a human, is capable of providing (directly or indirectly) the biologically active metabolite or residue thereof. Accordingly, for example, provided herein are pharmaceutically acceptable salts of polypeptides, prodrugs and pharmaceutically acceptable salts of such prodrugs, and other bioequivalents. The term "prodrug" indicates a therapeutic agent that is prepared in an inactive form and is converted to an active form (i.e., drug) within the body or cells thereof by the action of endogenous enzymes or other chemicals and/or conditions. The term "pharmaceutically acceptable salts" refers to physiologically and pharmaceutically acceptable salts of the polypeptides described herein (i.e., salts that retain the desired biological activity of the parent polypeptide without imparting undesired toxicological effects). Examples of pharmaceutically acceptable salts include, but are not limited to, salts formed with cations (e.g., sodium, potassium, calcium, or polyamines such as spermine); acid addition salts formed with inorganic acids (e.g., hydrochloric acid, hydrobromic acid, sulfuric acid, phosphoric acid, or nitric acid); and salts formed with organic acids (e.g., acetic acid, citric acid, oxalic acid, palmitic acid, or fumaric acid).

Pharmaceutical compositions containing the polypeptides described herein also can incorporate penetration enhancers that promote the efficient delivery of polypeptides to the skin of animals. Penetration enhancers can enhance the diffusion of both lipophilic and non-lipophilic drugs across cell membranes. Penetration enhancers can be classified as belonging to one of five broad categories, i.e., surfactants (e.g., sodium lauryl sulfate, polyoxyethylene-9-lauryl ether and polyoxyethylene-20-cetyl ether); fatty acids (e.g., oleic acid, lauric acid, myristic acid, palmitic acid, and stearic acid); bile salts (e.g., cholic acid, dehydrocholic acid, and deoxycholic acid); chelating agents (e.g., disodium ethylenediaminetetraacetate, citric acid, and salicylates); and non-chelating non-surfactants (e.g., unsaturated cyclic ureas). Alternatively, inhibitory polypeptides can be delivered via iontophoresis, which involves a transdermal patch with an electrical charge to "drive" the polypeptide through the dermis.

In some embodiments, a pharmaceutical composition can contain (a) one or more polypeptides and (b) one or more other agents that function by a different mechanism. For example, anti-inflammatory drugs, including but not limited to nonsteroidal anti-inflammatory drugs and corticosteroids, and antiviral drugs, including but not limited to ribivirin, vidarabine, acyclovir and ganciclovir, can be included in compositions as provided herein. Other non-polypeptide agents (e.g., chemotherapeutic agents) also are within the scope of the compositions provided herein. Such combined compounds can be used together or sequentially.

Compositions additionally can contain other adjunct components conventionally found in pharmaceutical compositions. Thus, the compositions also can include compatible, pharmaceutically active materials such as, for example, antipruritics, astringents, local anesthetics or anti-inflammatory agents, or additional materials useful in physically formulating various dosage forms of the compositions provided herein, such as dyes, flavoring agents, preservatives, antioxidants, opacifiers, thickening agents and stabilizers. Furthermore, the composition can be mixed with auxiliary agents, e.g., lubricants, preservatives, stabilizers, wetting agents, emulsifiers, salts for influencing osmotic pressure, buffers, colorings, flavorings, and aromatic substances. When added, however, such materials should not unduly interfere with the biological activities of the polypeptide components within the compositions provided herein. The formulations can be sterilized if desired.

Pharmaceutical formulations, which can be presented conveniently in unit dosage form, can be prepared according to conventional techniques well known in the pharmaceutical industry. Such techniques can include the step of bringing into association the active ingredients (e.g., the C_{H}2-C_{H}3 binding polypeptides provided herein) with the desired pharmaceutical carrier(s) or excipient(s). Typically, the formulations can be prepared by uniformly and bringing the active ingredients into intimate association with liquid carriers or finely divided solid carriers or both, and then, if necessary, shaping the product. Formulations can be sterilized if desired, provided that the method of sterilization does not interfere with the effectiveness of the polypeptide contained in the formulation.

The compositions provided herein can be formulated into any of many possible dosage forms such as, but not limited to, tablets, capsules, liquid syrups, soft gels, suppositories, and enemas. The compositions also can be formulated as suspensions in aqueous, non-aqueous or mixed media. Aqueous suspensions further can contain substances that increase the viscosity of the suspension including, for example, sodium carboxymethylcellulose, sorbitol, and/or dextran. Suspensions also can contain stabilizers.

C_{H}2-C_{H}3 binding polypeptides provided herein can be combined with packaging material and sold as kits for reducing Fc-mediated immune complex formation. Components and methods for producing articles of manufacture are well known. The articles of manufacture may combine one or more of the polypeptides and compounds set out in the above sections. In addition, the article of manufacture further may include, for example, buffers or other control reagents for reducing or monitoring reduced immune complex formation. Instructions describing how the polypeptides are effective for reducing Fc-mediated immune complex formation can be included in such kits.

### 6. Methods for using C_{H}2-C_{H}3 binding polypeptides to inhibit Fc-mediated immune complex formation

C_{H}2-C_{H}3 binding polypeptides can be used in *in vitro* assays of Fc-mediated immune complex formation. Such methods are useful to, for example, evaluate the ability of a C_{H}2-C_{H}3 cleft-binding polypeptide to block Fc-mediated immune complex formation. *In vitro* methods can include, for example, contacting an immunoglobulin molecule (e.g., an antigen bound immunoglobulin molecule) with an effector molecule (e.g., mClq, sClq, an FcR, FcRn, or another antibody) in the presence and absence of a polypeptide provided herein, and determining the level of immune complex formation in each sample. Levels of immune complex formation can be evaluated by, for example, polyacrylamide gel electrophoresis with Coomassie blue or silver staining, or by co-immunoprecipitation. Such methods include those known to persons of ordinary skill in the art. Methods provided herein also can be used to inhibit immune complex formation in a subject, and to treat an autoimmune disease in a subject by inhibiting Fc-mediated immune complex formation in. Such methods can involve, for example, administering any of the polypeptides provided herein, or a composition containing any of the polypeptides provided herein, to a subject. For example, a method can include administering to an individual a composition containing a polypeptide that includes the amino acid sequence Cys-Ala-Trp-His-Leu-Gly-Glu-Leu-Val-Trp-Cys-Thr (SEQ ID NO:10). Alternatively, a method can include administering to a subject a polypeptide that contains the amino acid sequence Asp-Cys-Ala-Trp-His-Leu-Gly-Glu-Leu-Val-Trp-Cys-Thr (SEQ ID NO:2), or Ala-Pro-Pro-Asp-Cys-Ala-Trp-His-Leu-Gly-Glu-Leu-Val-Trp-Cys-Thr (SEQ ID NO:16).

Methods provided herein can be used to treat a subject having, for example, PD, ALS, or AD. These conditions and the involvement of Fc-mediated immune complex formation are described herein. All of these neurodegenerative diseases are characterized by aggregation of intracellular proteins/inclusion bodies, abnormal microtubles, and activated microglia (CNS macrophages) that usually precede the onset of selective neuron loss and the onset of clinical symptoms for each of these diseases. ALS is characterized by the binding of immune complexed IgG Fc to an enzyme known as superoxide dismutase-1 (SOD1), aggregation of cellular proteins, abnormal microtubules, activation of microglia, and finally motor neuron death. PD is characterized by the binding of immune complexed IgG Fc to α-synuclein, aggregation of cellular proteins, abnormal microtubules, activation of microglia, and finally SN TH+ cell death. AD is characterized by the binding of immune complexed IgG Fc to tau proteins/microtubules, abnormal microtubule formation/aggregation, activation of microglia, and selective death of cholinergic cortical neurons. See, e.g., Henkel et al. (2004) Ann. Neurol. 55:221-235; Morrison et al. (2000) Exp. Neurol. 165:207-220; Orr et al. (2005) Brain 128:2665-2674; and Bouras et al. (2005) Brain Res. Brain Res. Rev. 48:477-487. This suggests that although the specific neurons are affected and different proteins are involved in these three diseases, the basic neuroimmunopathology may be similar (Couillard et al. (1998) Proc. Natl. Acad. Sci. USA 95:9626-9630; and Trojanowski et al. (1993) Brain Pathol. 3:45-54).

Methods also can include steps for identifying a subject in need of such treatment and/or monitoring treated individuals for a reduction in symptoms or levels of immune complex formation. For example, ALS can be monitored by measuring MCP-1 levels in the CNS, by electromyography, or by any other objective or subjective test (e.g., evaluation by a neurologist or other doctor) useful for measuring the progress or decline of an individual diagnosed with ALS.

*Parkinson's disease -* The clinical symptoms of PD result from the death of dopaminergic neurons in a section of the brain known as the substantia nigra (SN). An over responsive immune system may play a role in perpetuating PD by producing cytokines (e.g., interleukin-1 and tumor necrosis factor) in response to the initial damage, which can further injure cells in the brain. Immunoglobulins from PD individuals have been shown to contribute to the pathogenesis of SN cells (Chen et al. (1998) Arch. Neurol. 55:1075-1080). FcγRs appear to be essential in murine models of PD induced by the passive transfer of human PD IgG, as knockout of FcγRs can protect mice from both microglial activation and dopamine cell death (He et al. (2002) Exp. Neurol. 176:322-327; and Le et al. (2001) J. Neurosci. 21:8447-8455). Humoral (antibody) mediated immunity has been implicated in the immunopathogenesis of PD. Activated microglia express activating FcγR in both genetic and idiopathic (sporadic) PD, consistent with activation of microglia FcγR by neuronal IgG, predominantly IgG1 (Orr et al., *supra*). Further, SN cells selectively die in subjects with PD due to the accumulation of α-synuclein protein aggregates, which cause microtubule pathology. α-synuclein colocalizes with SN-specific IgG1, indicating that α-synuclein may interact with immune complexed IgG.

*Amyotrophic lateral sclerosis -* ALS is a devastating disease with upper and lower motor neuron (MN) degeneration, which ultimately leads to death due to respiratory failure within one to five years. IgG immune complexes from ALS patients can induce ALS like lesions in mice. IgG FcγRs appear to be essential in the immunopathology of ALS (Mohamed et al. (2002) J. Neurosci. Res. 69:110-116; and Engelhardt et al. (2005) Acta Neurol. Scand. 112:126-133; and Zhao et al. (2003) J. Neuropathol. Exp. Neurol. 63:964-977). Less than ten percent of ALS cases have a familial (inherited) "fALS" basis. Approximately two percent of all human ALS cases are due to over one hundred known mutations in the gene encoding SOD1 (Alexianu et al. (2001) Neurol. 57:1282-1289). Mice transgenic for human SOD1 mutations, such as SOD1 G93A, exhibit immune reactivity with the appearance of increased IgG, FcγR and activated microglia preceding the loss of motor neurons (MN) and the onset of clinical signs consistent with the clinical and histopathological progression of human ALS. More than ninety percent of all ALS cases have a sporadic "sALS" basis, with no known inherited basis or any known mutation related to either SOD1 or microtubules (Valentine et al. (2005) Ann. Rev. Biochem. 74:563-593; and Garcia et al. (2006) Neurobiol. Dis. 21:102-109).

Dendritic cells, monocyte chemoattractant protein-1 (MCP-1), and activated microglia/microphages have been found in the spinal cords of fALS and sALS (Henkel et al., *supra*). Immune complexed IgG (but not monomeric, non-immune complexed IgG, or F(ab')₂ fragments of IgG) increased MCP-1 mRNA expression ten fold through the binding and activation of immune complexed IgG FcγR (Hora et al. (1992) Proc. Natl. Acad. Sci. USA 89:1745-1749), suggesting that immune complexed IgG activate MCP-1 via FcγR (most likely on surrounding microglia) in both fALS and sALS cases.

*Alzheimer's disease -* Traditional scientific view is that immunoglobulins, including IgG, cannot reach the brain due to exclusion of IgG to the brain through the blood-brain barrier (BBB). With inflammatory neurological disease or as a result of normal brain aging, however, IgG can enter the brain across a dysfunctional BBB. IgG Fc can activate microglia, which have been implicated in the immunopathogenesis of AD through cellular FcγRs (e.g., FcγRI and FcγRIII). Immunocytochemical experiments have shown FcγRI in the same pyramidial neurons that are IgG-immunoreactive in the aged brain, suggesting a role of these receptors in intraneuronal penetration of IgG Fc. Intraneuronal IgG Fc has been shown to bind to tau proteins. Since tau proteins have been shown to been essential in the correct polymerization of microtubles, the binding of IgG Fc (but not IgG Fab fragments) to tau proteins and to microtubles themselves may cause abnormal polymerization of microtubules. Thus, IgG Fc intraneuronal penetration may participate in the early stages of neurodegeneration in vulnerable subsets of cortical neurons (Reiderer et al. (2003) NeuroReport 14:117-121; Bouras et al., *supra*; and Engelhardt et al. (2000) Arch. Neurol. 57:681-686). The role of FcγR in AD immunopathology can be found in the activation of FcγR+ microglia in senile plaques in AD (Peress et al. 1993 J. Neuroimmunol. 48:71-79).

The invention will be further described in the following examples, which do not limit the scope of the invention described in the claims.

### EXAMPLES

### Example 1 - In vitro assays for measuring ligand binding to the C_{H}2-C_{H}3 cleft

*In vitro* assays involving enzyme-linked immunosorbent assay (ELISA) and double immunodiffusion techniques were used to demonstrate competitive inhibition of immune complexed IgG Fc binding to factors such as FcRs, FcRn, C1q, α-synuclein, SOD1, and tau proteins by polypeptides and compounds provided herein.

In a standard ELISA, an antigen is immunoadsorbed onto a plastic microwell. After blocking and washing, a primary antibody with specificity directed toward the antigen is added to the microwell. After another wash phase, a secondary antibody that is directed toward the primary antibody and conjugated to an enzyme marker such as horseradish peroxidase (HRP) is added to the microwell. Following another wash cycle, the appropriate enzyme substrate is added. If an antigen/primary antibody/secondary antibody/HRP conjugate is formed, the conjugated enzyme catalyzes a colorimetric chemical reaction with the substrate, which is read with a microplate reader or spectrophotometer. By standardizing the levels of antigen and secondary antibody/HRP conjugate, a titer of the primary antibody (the variable) is established. In a standard ELISA system, the primary antibody binds to the antigen through its complementarity determining regions (CDR) located in the Fab arms. Likewise, the secondary antibody/HRP conjugate binds to the primary antibody via its CDR Fab region. Because the HRP is conjugated to the Fc region of the secondary antibody, direct Fc binding is very limited or abrogated.

For this reason, a "reverse ELISA" technique was used to assess binding of the Fc region to ligands that bind to immune complexed IgG Fc. In these assays, the enzyme (HRP) was not covalently conjugated to the Fc portion of the secondary antibody. Rather, a preformed immune complex of peroxidase-rabbit (or mouse) anti-peroxidase IgG ("PAP" complex) was used. Thus, HRP served both as the antigen and the enzyme marker, but did not block the Fc region. In the reverse ELISA system, an Fc C_{H}2-C_{H}3 cleft binding ligand (e.g., purified human C1q) was bound to microwell plates. In the absence of competitor, PAP complexes bound to the immobilized ligand and the reaction between HRP and its substrate produced a signal. This signal was reduced by polypeptides and compounds, such as those provided herein, that inhibited PAP binding to the immobilized ligand.

### Example 2 - Inhibition of C1q binding

PAP complexes were formed by mixing 2 µl of rabbit anti-peroxidase (Sigma Chemicals, St. Louis, MO) with 50 µl of peroxidase (Sigma-Aldrich, St. Louis, MO) in 1 ml distilled water. PAP (100 µl) were pre-incubated with 100 µl of peptide or human C1q (Quidel Corp., San Diego, CA) for one hour. The C1q/PAP and peptide/PAP mixtures (100 µl) were then incubated with C1q coated plates for 30 minutes. After washing, plates were incubated with 2-2'-azinobis-3-ethylbenzthiazoline-6-sulfonate (ATBS; Quidel Corp.) for 15 minutes and read at 405 nm. Results are shown in Table 1.

**Table 1**

| **Peptide** | **SEQ ID NO:** | **OD 405 nm** |
|---|---|---|
| DCAWHLGELVWCT | 2 | 1.100 |
| APPCARHLGELVWCT | 14 | 0.567 |
| DCAFHLGELVWCT | 3 | 0.859 |
| APPDCAWHLGELVWCT | 16 | 0.389 |
| APPCAFHLGELVWCT | 15 | 0.983 |
| APPCAWHLGELVWCT | 13 | 1.148 |
| C1q (negative control) | - | 0.337 |
| Positive control | - | 2.355 |

APPDCAWHLGELVWCT (SEQ ID NO:16) resulted in the greatest inhibition of C1q binding, almost equaling C1q itself. Peptide APPCARHLGELVWCT (SEQ ID NO:14) gave the next best result.

### Example 3 - Inhibition of FcR binding

Once the reverse ELISA protocol was established using the C1q assay, the assay was redesigned using FcγIIa, FcγIIb and FcγIII in place of C1q. Highly purified FcγIIa, FcγIIb and FcγIII were immunoadsorbed onto plastic microwells. After optimizing the FcγR reverse ELISA system, competitive inhibition experiments using polypeptides as described herein were conducted to investigate their ability to inhibit binding of immune complexes to purified FcγR.

Falcon microtiter plates were coated with 1:10 dilutions of highly purified FcγIIa, FcγIIb and FcγIII and incubated for 24 hours. The plates were washed and then blocked with 5X BSA blocking solution (Alpha Diagnostic International, San Antonio, Texas) for 24 hours. PAP immune complexes were formed as described in Example 2. PAP (100 µl) were pre-incubated with 100 µl of peptide for one hour. PAP/peptide mixtures were added to the FcγR coated plates and incubated for one hour. After washing, plates were incubated with ABTS substrate for 15 minutes and read at 405 nm. Results are shown in Table 2.

**Table 2**

| **Peptide** | **SEQ ID NO:** | **FcγIIa** | **FcγIIb** | **FcγIII** |
|---|---|---|---|---|
| DCAWHLGELVWCT | 2 | 0.561 | 0.532 | 0.741 |
| APPCARHLGELVWCT | 14 | 0.956 | 0.768 | 0.709 |
| DCAFHLGELVWCT | 3 | 0.660 | 0.510 | 0.810 |
| APPDCAWHLGELVWCT | 16 | 0.509 | 0.496 | 0.670 |
| APPCAFHLGELVWCT | 15 | 0.605 | 0.380 | 0.880 |
| APPDCAWHLGELVWCT | 13 | 0.658 | 0.562 | 0.530 |
| Positive Control | - | 1.599 | 1.394 | 1.588 |

Peptide APPDCAWHLGELVWCT (SEQ ID NO:16) resulted in the greatest inhibition of FcR binding to PAP, followed by peptide DCAWHLGELVWCT (SEQ ID NO:2).

### Example 4 - Inhibition of immune complexed IgG binding to wt and mutant SOD1

PAP complexes were formed as described in Example 2. PAP (100 µl) were pre-incubated with 100 µl of peptide for one hour, and control (200 µl of PAP) also was incubated for one hour. 100 µl of either PAP or PAP/peptide was added to Falcon microtiter plates that had been coated for 24 hours with either 0.8 µg/ml human wtSOD1 (Sigma-Aldrich) or apo (non Cu, Zn containing) monomeric mutant SOD1 C57S (mSOD1C57S). The PAP or peptide/PAP mixtures (100 µl) were added after one hour to either wt SOD1 or mSOD1C57S coated plates for 60 minutes. After washing, plates were incubated with ATBS for 15 minutes and read at 405 nm. Results are shown in Table 3.

**Table 3**

| **Peptide** | **SEQ ID NO:** | **wt SOD1** | **mSOD1C57S** |
|---|---|---|---|
| DCAWHLGELVWCT | 2 | 0.292 | 0.592 |
| APPDCAWHLGELVWCT | 16 | 0.277 | 0.226 |
| Positive Control | - | 2.900 | 2.924 |

Peptide APPDCAWHLGELVWCT (SEQ ID NO:16) resulted in the greatest inhibition of wt SOD1 or mSOD1C57S binding to PAP, followed by peptide DCAWHLGELVWCT (SEQ ID NO:2).

Because SOD1 can use hydrogen peroxide and ABTS as substrates under certain conditions (Elam et al. (2003) J. Biol. Chem. 278:21032-21039; and Yim et al. (1993) J. Biol. Chem. 268:4099-4105), 0.05 mg/ml wt SOD1 or 0.050 mg/ml peroxidase (Sigma-Aldrich) were added to the ABTS substrate used in the SOD immune complex binding assay described above. The plates were read at 405 nm after a five-minute incubation. The results are shown in Table 4.

**Table 4**

| **Enzyme** | **0.05 mg/ml wt SOD1** | **0.050 mg/ml peroxidase** | **Substrate alone** |
|---|---|---|---|
| Hydrogen peroxide/ABTS substrate | 0.063 | 3.432 | 0.061 |

Taken together, these data indicate that immune complexed IgFc binding to both wtSOD1 and mSOD1C57S was inhibited by peptides 2 and 16, with peptide 16 giving the best inhibition. Since peptides 2 and 16 bind only to the IgG Fc C_{H}2-C_{H}3 cleft, it follows that immune complexed IgG Fc C_{H}2-C_{H}3 cleft binds to both wtSOD1 and mSOD1S57S. As shown in Table 4, 100X of wt SOD had no effect on the OD readings of the ABTS substrate used in the experiments summarized in Table 3.

### Example 5 - Inhibition of immune complexed IgG binding to wt α-synuclein

PAP complexes were formed mixing 2 µl of rabbit anti-peroxidase (Sigma) with 50 µl of peroxidase (Sigma) in 1 ml distilled water. PAP (100 µl) were pre-incubated with 100 µl of peptide for one hour. Control PAP (200 µl) also was incubated for one hour. 100µl of either PAP or PAP/peptide was added to Falcon microtiter plates that had been coated for 24 or 48 hours with 167 µg/ml human wt α-synuclein (Sigma-Aldrich), and the plates were incubated for 60 minutes. After washing, plates were incubated with ATBS for 30 minutes and then read at 405 nm. Results are shown in Table 5.

**Table 5**

| **Peptide** | **SEQ ID NO:** | **Wt α-synuclein** |
|---|---|---|
| DCAWHLGELVWCT (24 h) | 2 | 0.111 |
| APPDCAWHLGELVWCT (24 h) | 16 | 0.088 |
| Positive control | - | 1.433 |
| ABTS substrate only | | 0.061 |
| DCAWHLGELVWCT (48h) | 2 | 0.152 |
| APPDCAWHLGELVWCT (48h) | 16 | 0.136 |
| α-synuclein positive control (48h) | - | 2.934 |

Peptide APPDCAWHLGELVWCT (SEQ ID NO:16) resulted in the greatest inhibition of α-synuclein binding to PAP, followed by peptide DCAWHLGELVWCT (SEQ ID NO:2).

### Example 6 - Inhibition of ALS antibodies in an in vitro system

To determine whether blocking FcRs and C1q with the inhibitors described herein can reduce the MN cell damage and MN cell death associated with ALS, cell cultures containing microglia and motor neurons (MN) are prepared as described in Zhao et al. (J. Neuropath. Exp. Neurol. (2004) 63:964-977). ALS IgG (1 or 2 µg/ml) is added to the microglia/MN cell cultures with and without the FcR and C1q binding inhibitors described herein (e.g., polypeptides having the amino acid sequences set forth in SEQ ID NOS:2 and 16). A microglial activation assay is performed by measuring the amount of the pro-inflammatory cytokine, TNF-α. Immunocytochemical MN staining is done using anti-p75 and/or anti-CFAp antibody to determine MN survival with and without the immune complexed IgG inhibitors. Glutamate, known to be cytotoxic for MN, is measured using high-performance liquid chromatography.

### Example 7 - Inhibition of ALS immune complexed IgG Fc in an in vivo model

IgG (40 mg) prepared from ALS patients (Mohamed et al. (2002) J. Neurosci. Res. 69:110-116) is injected intraperitoneally (i.p.) into C57B1/6 X 129SvEv mice aged 13-17 weeks, with and without i.p. injection of the polypeptide inhibitors described herein. Ultrastructural detection of ALS IgG in MN, intracellular calcium release, and actylcholine release at the neuromuscular junctions is used to monitor ALS-like symptoms in control and treated mice. Alternatively, FcγRIIb knockout mice are used. Experiments utilizing these mice have shown that clinical signs of either rheumatoid arthritis (RA) or systemic lupus erythematosus (SLE) can be induced by passive administration of human RA or SLE sera (Petkova et al. (2006) J. Exp. Med. 203(2):275-280). Thus, administration of ALS positive sera to FcγRIIbR knockout mice is used to passively induce ALS, with and without the inhibitors described herein.

### Example 8 - Inhibition of PD immune complexed IgG Fc in an in vitro model

Experiments are conducted to determine whether the inhibitors described herein can prevent the cellular destruction of dopaminergic cells that is associated with the clinical immunopathology of PD. The dopaminergic cell line MES 23.5 is prepared with purified mouse microglia (Le et al. (2001) J. Neurosci. 21:8447-8455). Purified PD immune complexed IgG is prepared and added to the cell cultures with and without the FcR inhibitors described herein. Inhibition of α-synuclein binding to immune complexed IgG Fc also is measured according to the methods described in Example 5.

### Example 9 - Inhibition of PD immune complexe IgG Fc in an in vivo model

*In vivo* experiments are conducted to determine whether the inhibitors described herein can prevent the binding of PD IgG to microglial FcγR and thus prevent destruction of SN dopaminergic cells, the primary pathological lesion associated with PD. PD IgG is purified as described (He et al., *supra*) and 20 µl is stereotactically injected into the mouse SN. FcγIIb knockout mice also are used as an *in vivo* model of passively transferred PD, with and without the inhibitors.

### Example 10 - Inhibition of AD IgG mediated injury to cholinergic neurons in an in vivo model using FcγR inhibitors

AD is a progressive neurodegenerative disease characterized by loss of cholinergic neurons (CN) in the basal forebrain. Stereotaxic injection of AD IgG into the basal forebrain of adult female Sprague-Dawley rats resulted in a significant reduction in the ChAT-immunostained CN cells that are characteristic of CN degeneration in human AD patients (Engelhardt et al. (2000) *supra*). Thus, *in vivo* experiments are conducted to determine whether the FcγR inhibitors described herein can prevent activation of FcγR microglia and internalization of IgG Fc to CN cells, which leads to the degenerative immnopathology seen in AD. FcγIIb knockout mice also are be used as an *in vivo* model of passively transferred AD, with and without the inhibitors.

### Example 11 - Inhibition of immune complexed IgG binding to wt tau

PAP complexes were formed by mixing 2 µl of rabbit anti-peroxidase with 50 µl of peroxidase in 1 ml distilled water. PAP (100 µl) were pre-incubated with 100 µl of peptide for one hour. Control PAP (200 µl) also was incubated for one hour. 100 µl of either PAP or PAP/peptide was added to Falcon microtiter plates that had been coated for 24 hours with 16.7 µg/ml human wt tau protein (441 residues; Sigma-Aldrich). The PAP or peptide/PAP mixtures (100 µl) were added after one hour to tau coated plates and incubated for 60 minutes. After washing, plates were incubated with ATBS for 15 minutes, and read at 405 nm. Results are shown in Table 6.

**Table 6**

| **Peptide** | **SEQ ID NO:** | **Wt tau protein** |
|---|---|---|
| DCAWHLGELVWCT | 2 | 0.322 |
| APPDCAWHLGELVWCT | 16 | 0.059 |
| Positive control | - | 0.695 |
| Negative control (substrate only) | - | 0.060 |

Peptide APPDCAWHLGELVWCT (SEQ ID NO:16) resulted in the greatest inhibition of human wt tau protein binding to PAP, followed by peptide DCAWHLGELVWCT (SEQ ID NO:2).

### Example 12 - Inhibition of immune complexed IgG binding to wt β-amyloid peptide

Different fragments of the Aβ peptide contribute to the amyloid plaques pathognomic for AD, and these Aβ fragments appear to stimulate microglial activation and subsequent AD neuropathology. Thus, experiments were conducted to test Aβ 1-40 for binding to immune complexes, and to determine whether the inhibitors described herein can inhibit binding of PAP immune complexes to Aβ peptide. PAP complexes were prepared by mixing 2 µl of rabbit anti-peroxidase with 50 µl of peroxidase in 1 ml distilled water. PAP (100 µl) were pre-incubated with 100 µl of peptide for one hour. Control PAP (200 µl) also were incubated for one hour, and 100 µl of either PAP or PAP/peptide was added to Falcon microtiter plates that had been coated for 24 hours with 33 µg/ml human wt β amyloid peptide 1-40 (amyloid precursor protein (APP) fragment 1-40; Sigma-Aldrich) having the sequence Asp-Ala-Glu-Phe-Arg-His-Asp-Ser-Gly-Tyr-Glu-Val-His-His-Gln-Lys-Leu-Val-Phe-Phe-Ala-Glu-Asp-Val-Gly-Ser-Ans-Lys-Gly-Ale-Ile-Ile-Gly-Leu-Met-Val-Gly-Gly-Val-Val (SEQ ID NO:39). The PAP or peptide/PAP mixtures were incubated on the β-amyloid peptide coated plates for 60 minutes. After washing, plates were incubated with ATBS for 15 minutes and read at 405 nm. Results are shown in Table 7.

**Table 7**

| **Peptide** | **SEQ ID NO:** | **Wt 1-40 β-amyloid peptide** |
|---|---|---|
| DCAWHLGELVWCT | 2 | 0.151 |
| APPDCAWHLGELVWCT | 16 | 0.885 |
| Positive control | - | 3.278 |
| Negative control (substrate only) | - | 0.061 |

Peptide DCAWHLGELVWCT (SEQ ID NO:2) resulted in the greatest inhibition of human wt β amyloid peptide binding to PAP, followed by peptide APPDCAWHLGELVWCT (SEQ ID NO:16).

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

## Claims

1. A composition comprising a purified polypeptide, said polypeptide comprising the amino acid sequence (Xaa₁)ₙ-Cys-Ala-Xaa₂-His-Leu-Gly-Glu-Leu-Val-Trp-Cys-Thr-(Xaa₃)ₙ (SEQ ID NO:35), wherein Xaa₁ is any amino acid, Xaa₂ is Arg, Trp, 5-HTP, Tyr, or Phe, Xaa₃ is any amino acid, and n is 0, 1, 2, 3, 4, or 5 for use in inhibiting immune complex formation in a subject, and wherein said immune complex formation is associated with amyotrophic lateral sclerosis (ALS) or Alzheimer's disease (AD).

2. The composition of claim 1, wherein said polypeptide inhibits binding of ALS IgG Fc to FCγI, FcγIIa, FcγIIb, FcγIIIa, FcγIIIb, FcRn, mC1q, sC1q, wild type SOD1 or mutant SOD1.

3. The composition of claim 1, wherein said polypeptide inhibits binding of AD IgG Fc to FCγI, FcγIIa, FcγIIb, FcγIIIa, FcγIIIb, FcRn, mC1q, sC1q, tau protein, β-amyloid peptide, microtubules, or aggregates of tau protein and microtubules.

4. The composition of any one of claims 1 to 3, wherein said polypeptide comprises a terminal-stabilizing group.

5. The composition of claim 4, wherein said terminal stabilizing group is at the amino terminus of said polypeptide and is a tripeptide having the amino acid sequence Xaa-Pro-Pro, wherein Xaa is any amino acid.

6. The composition of claim 5, wherein Xaa is Ala.

7. The composition of claim 1, wherein said polypeptide comprises an Asp at the amino terminus of said amino acid sequence.

8. The composition of any one of claims 1 to 7, wherein said polypeptide has a length of about 10 to about 50 amino acids.

9. The composition of any one of claims 1 to 8, wherein said polypeptide comprises the amino acid sequence Asp-Cys-Ala-Trp-His-Leu-Gly-Glu-Leu-Val-Trp-Cys-Thr (SEQ ID NO:2).

10. The composition of any one of claims 1 to 9, wherein said polypeptide comprises the amino acid sequence Ala-Pro-Pro-Asp-Cys-Ala-Trp-His-Leu-Gly-Glu-Leu-Val-Trp-Cys-Thr (SEQ ID NO: 16).

11. Use of a polypeptide in the manufacture of a medicament for treating ALS or AD, wherein said polypeptide comprises the amino acid sequence (Xaa₁)ₙ-Cys-Ala-Xaa₂-His-Leu-Gly-Glu-Leu-Val-Trp-Cys-Thr-(Xaa₃)ₙ (SEQ ID NO:35), wherein Xaa₁ is any amino acid, Xaa₂ is Arg, Trp, 5-HTP, Tyr, or Phe, Xaa₃ is any amino acid, and n is 0, 1, 2, 3, 4, or 5.

12. The use of claim 11, wherein said polypeptide inhibits binding of ALS IgG Fc to FCγI, FcγIIa, FcγIIb, FcγIIIa, FcγIIIb, FcRn, mC1q, sC1q, wild type SOD1 or mutant SOD1.

13. The use of claim 11, wherein said polypeptide inhibits binding of AD IgG Fc to FCγI, FcγIIa, Fcyllb, FcγIIIa, FcγIIIb, FcRn, mClq, sClq, tau protein, β-amyloid peptide, microtubules, or aggregates of tau protein and microtubules.

14. The use of any one of claims 11 to 13, wherein said polypeptide comprises a terminal-stabilizing group.

15. The use of claim 14, wherein said terminal stabilizing group is at the amino terminus of said polypeptide and is a tripeptide having the amino acid sequence Xaa-Pro-Pro, wherein Xaa is any amino acid.

16. The use of claim 15, wherein Xaa is Ala.

17. The use of claim 11, wherein said polypeptide comprises an Asp at the amino terminus of said amino acid sequence.

18. The use of any one of claims 11 to 17, wherein said polypeptide has a length of about 10 to about 50 amino acids.

19. The use of any one of claims I 1 to 18, wherein said polypeptide comprises the amino acid sequence Asp-Cys-Ala-Trp-His-Leu-Gly-Glu-LeuVal-Trp-Cys-Thr (SEQ ID NO:2).

20. The composition of any one of claims 11 to 19, wherein said polypeptide comprises the amino acid sequence Ala-Pro-Pro-Asp-Cys-Ala-Trp-His-Leu-Gly-Glu-Leu-Val-Trp-Cys-Thr (SEQ ID NO:16).

## Patentansprüche

1. Zusammensetzung, umfassend ein gereinigtes Polypeptid, wobei das Polypeptid die Aminosäuresequenz (Xaa₁)ₙ-Cys-Ala-Xaa₂-His-Leu-Gly-Glu-Leu-Val-Trp-Cys-Thr-(Xaa₃)ₙ (SEQ ID NO: 35) umfasst, wobei Xaa₁ für eine beliebige Aminosäure steht, Xaa₂ für Arg, Trp, 5-HTP, Tyr oder Phe steht, Xaa₃ für jede Aminosäure steht, und n für 0, 1, 2, 3, 4 oder 5 steht, zur Verwendung bei der Inhibition der Immunkomplexbildung in einem Subjekt, und wobei die Immunkomplexbildung mit amyotropher Lateralsklerose (ALS) oder Alzheimer-Krankheit (AD) assoziiert ist.

2. Zusammensetzung nach Anspruch 1, wobei das Polypeptid die Bindung von ALS-IgG-Fc an FcγI, FcγIIa, FcγIIb, FcγIIIa, FcγIIIb, FcRn, mC1q, sC1q, Wildtyp-SOD1 oder mutiertes SOD1 inhibiert.

3. Zusammensetzung nach Anspruch 1, wobei das Polypeptid die Bindung von AD-IgG-Fc an FcγI, FcγIIa, FcγIIb, FcγIIIa, FcγIIIb, FcRn, mC1q, sC1q, tau-Protein, β-Amyloidpeptid, Mikrotubuli oder Aggregate aus tau-Protein und Mikrotubuli inhibiert.

4. Zusammensetzung nach einem der Ansprüche 1 bis 3, wobei das Polypeptid eine terminal-stabilisierende Gruppe umfasst.

5. Zusammensetzung nach Anspruch 4, wobei sich die terminal stabilisierende Gruppe am Aminoterminus des Polypeptids befindet und ein Tripeptid ist, das die Aminosäuresequenz Xaa-Pro-Pro aufweist, wobei Xaa für eine beliebige Aminosäure steht.

6. Zusammensetzung nach Anspruch 5, wobei Xaa für Ala steht.

7. Zusammensetzung nach Anspruch 1, wobei das Polypeptid eine Asp am Aminoterminus der Aminosäuresequenz umfasst.

8. Zusammensetzung nach einem der Ansprüche 1 bis 7, wobei das Polypeptid eine Länge von etwa 10 bis etwa 50 Aminosäuren aufweist.

9. Zusammensetzung nach einem der Ansprüche 1 bis 8, wobei das Polypeptid die Aminosäuresequenz Asp-Cys-Ala-Trp-His-Leu-Gly-Glu-Leu-Val-Trp-Cys-Thr (SEQ ID NO: 2) umfasst.

10. Zusammensetzung nach einem der Ansprüche 1 bis 9, wobei das Polypeptid die Aminosäuresequenz Ala-Pro-Pro-Asp-Cys-Ala-Trp-His-Leu-Gly-Glu-Leu-Val-Trp-Cys-Thr (SEQ ID NO: 16) umfasst.

11. Verwendung eines Polypeptids bei der Herstellung eines Medikaments zur Behandlung von ALS oder AD, wobei das Polypeptid die Aminosäuresequenz (Xaa₁)ₙ-Cys-Ala-Xaa₂-His-Leu-Gly-Glu-Leu-Val-Trp-Cys-Thr-(Xaa₃)ₙ (SEQ ID NO:35) umfasst, wobei Xaa₁ für eine beliebige Aminosäure steht, Xaa₂ für Arg, Trp, 5-HTP, Tyr oder Phe steht, Xaa₃ für jede Aminosäure steht, und n für 0, 1, 2, 3, 4 oder 5 steht.

12. Verwendung nach Anspruch 11, wobei das Polypeptid die Bindung von ALS-IgG-Fc an FcγI, FcγIIa, FcγIIb, FcγIIIa, FcγIIIb, FcRn, mC1q, sC1q, Wildtyp-SOD1 oder mutiertes SOD1 inhibiert.

13. Verwendung nach Anspruch 11, wobei das Polypeptid die Bindung von AD-IgG-Fc an FcγI, FcγIIa, FcγIIb, FcγIIIa, FcγIIIb, FcRn, mC1q, sC1q, tau-Protein, β-Amyloidpeptid, Mikrotubuli oder Aggregate aus tau-Protein und Mikrotubuli inhibiert.

14. Verwendung nach einem der Ansprüche 11 bis 13, wobei das Polypeptid eine terminal-stabilisierende Gruppe umfasst.

15. Verwendung nach Anspruch 14, wobei sich die terminal stabilisierende Gruppe am Aminoterminus des Polypeptids befindet und ein Tripeptid ist, das die Aminosäuresequenz Xaa-Pro-Pro aufweist, wobei Xaa für eine beliebige Aminosäure steht.

16. Verwendung nach Anspruch 15, wobei Xaa für Ala steht.

17. Verwendung nach Anspruch 11, wobei das Polypeptid eine Asp am Aminoterminus der Aminosäuresequenz umfasst.

18. Verwendung nach einem der Ansprüche 11 bis 17, wobei das Polypeptid eine Länge von etwa 10 bis etwa 50 Aminosäuren aufweist.

19. Verwendung nach einem der Ansprüche 11 bis 18, wobei das Polypeptid die Aminosäuresequenz Asp-Cys-Ala-Trp-His-Leu-Gly-Glu-Leu-Val-Trp-Cys-Thr (SEQ ID NO: 2) umfasst.

20. Verwendung nach einem der Ansprüche 11 bis 19, wobei das Polypeptid die Aminosäuresequenz Ala-Pro-Pro-Asp-Cys-Ala-Trp-His-Leu-Gly-Glu-Leu-Val-Trp-Cys-Thr (SEQ ID NO: 16) umfasst.

## Revendications

1. Composition comprenant un polypeptide purifié, ledit polypeptide comprenant la séquence d'acide aminé (Xaa₁)ₙ-Cys-Ala-Xaa₂-His-Leu-Gly-Glu-Leu-Val-Trp-Cys-Thr-(Xaa₃)ₙ (SEQ. ID N°35), dans laquelle Xaa₁ est n'importe quel acide aminé, Xaa₂ est une Arg, Trp, 5-HTP, Tyr, ou Phe, Xaa₃ est n'importe quel acide aminé, et n est égal à 0, 1, 2, 3, 4 ou 5, à utiliser pour inhiber la formation d'un complexe immun chez un sujet, et dans laquelle ladite formation de complexe immun est associée à une sclérose latérale amyotrophique (ALS) ou à la maladie d'Alzheimer (AD).

2. Composition selon la revendication 1, dans laquelle ledit polypeptide inhibe la liaison d'IgG Fc de l'ALS à FCγI, FcγIIa, FcγIIb, FcγIIIa, FcγIIIb, FcRn, mClq, sClq, SOD1 de type sauvage ou SOD1 mutant.

3. Composition selon la revendication 1, dans laquelle ledit polypeptide inhibe la liaison d'IgG Fc d'AD à FCγI, FcγIIa, FcγIIb, FcγIIIa, FcγIIIb, FcRn, mClq, sClq, protéine tau, peptide β-amyloïde, microtubules, ou agrégats de protéine tau et de microtubules.

4. Composition selon l'une quelconque des revendications 1 à 3, dans laquelle ledit polypeptide comprend un groupe stabilisateur de terminaison.

5. Composition selon la revendication 4, dans laquelle ledit groupe stabilisateur de terminaison est sur la terminaison amino dudit polypeptide et est un tripeptide ayant la séquence d'acide aminé Xaa-Pro-Pro, dans laquelle Xaa est n'importe quel acide aminé.

6. Composition selon la revendication 5, dans laquelle Xaa est une Ala.

7. Composition selon la revendication 1, dans laquelle ledit polypeptide comprend une Asp à la terminaison amino de ladite séquence d'acide aminé.

8. Composition selon l'une quelconque des revendications 1 à 7, dans laquelle ledit polypeptide a une longueur d'environ 10 à environ 50 acides aminés.

9. Composition selon l'une quelconque des revendications 1 à 8, dans laquelle ledit polypeptide comprend la séquence d'acide aminé Asp-Cys-Ala-Trp-His-Leu-Gly-Glu-Leu-Val-Trp-Cys-Thr (SEQ. ID N°2).

10. Composition selon l'une quelconque des revendications 1 à 9, dans laquelle ledit polypeptide comprend la séquence d'acide aminé Ala-Pro-Pro-Asp-Cys-Ala-Trp-His-Leu-Gly-Glu-Leu-Val-Trp-Cys-Thr (SEQ. ID N°16).

11. Utilisation d'un polypeptide dans la fabrication d'un médicament pour traiter l'ALS ou l'AD, dans laquelle ledit polypeptide comprend la séquence d'acide aminé (Xaa₁)ₙ-Cys-Ala-Xaa₂-His-Leu-Gly-Glu-Leu-Val-Trp-Cys-Thr-(Xaa₃)ₙ (SEQ. ID N°35), dans laquelle Xaa₁ est n'importe quel acide aminé, Xaa₂ est une Arg, Trp, 5-HTP, Tyr ou Phe, Xaa₃ est n'importe quel acide aminé et n est égal à 0, 1, 2, 3, 4, ou 5.

12. Utilisation selon la revendication 11, dans laquelle ledit polypeptide inhibe la liaison d'IgG Fc d'ALS à FCγI, FcγIIa, FcγIIb, FcγIIIa, FcγIIIb, FcRn, mClq, sClq, SOD1 de type sauvage ou SOD1 mutant.

13. Utilisation selon la revendication 11, dans laquelle ledit polypeptide inhibe la liaison de l'IgG Fc de l'AD à FCγI, FcγIIa, FcγIIb, FcγIIIa, FcγIIIb, FcRn, mClq, sClq, protéine tau, peptide β-amyloïde, microtubules, ou agrégats de protéine tau et de microtubules.

14. Utilisation selon l'une quelconque des revendications 11 à 13, dans laquelle ledit polypeptide comprend un groupe stabilisateur de terminaison.

15. Utilisation selon la revendication 14, dans laquelle ledit groupe stabilisateur de terminaison est sur la terminaison amino dudit polypeptide et est un tripeptide ayant la séquence d'acide aminé Xaa-Pro-Pro, dans laquelle Xaa est n'importe quel acide aminé.

16. Utilisation selon la revendication 15, dans laquelle Xaa est une Ala.

17. Utilisation selon la revendication 11, dans laquelle ledit polypeptide comprend une Asp sur la terminaison amino de ladite séquence d'acide aminé.

18. Utilisation selon l'une quelconque des revendications 11 à 17, dans laquelle ledit polypeptide a une longueur d'environ 10 à environ 50 acides aminés.

19. Utilisation selon l'une quelconque des revendications 11 à 18, dans laquelle ledit polypeptide comprend la séquence d'acide aminé Asp-Cys-Ala-Trp-His-Leu-Gly-Glu-Leu-Val-Trp-Cys-Thr (SEQ. ID N°2).

20. Composition selon l'une quelconque des revendications 11 à 19, dans laquelle ledit polypeptide comprend la séquence d'acide aminé Ala-Pro-Pro-Asp-Cys-Ala-Trp-His-Leu-Gly-Glu-Leu-Val-Trp-Cys-Thr (SEQ. ID N°16).
